# EUROPEAN PATENT APPLICATION

(11) **EP 4 427 736 A2**
(43) Date of publication of application: **11.09.2024**
(21) Application number: 24159031.4
(22) Date of filing: 16.03.2018
(51) Int. Cl.: A61K 9/00

(54) **CHEWABLE GEL PRODUCTS FOR ACTIVE PHARMACEUTICAL INGREDIENTS**

(30) Priority: 20.03.2017 US 201762473763 P
(62) Divisional of application: 18770795.5
(71) Applicant: Bayer HealthCare LLC, Whippany, NJ 07981 (US)
(72) Inventor: Sirihorachai, Rachan, Whippany, 07981-0915 (US); Bradley, Reginald, Whippany, 07981-0915 (US); Rosar, Paul, Whippany, 07981-0915 (US); Ekpe, Anthony, Whippany, 07981-0915 (US); Joshi Charu, Whippany, 07981-0915 (US)
(74) Representative: BIP Patents

(57) **Abstract**

The present disclosure relates to chewable gel products containing a gel carrier and an active pharmaceutical ingredient (API), where the chewable gel product has an acceptable taste.

## Description

### CROSS-REFERENCE TO RELATED APPLICATIONS

This application claims the benefit of U.S. Provisional Application No. 62/473,763, filed March 20, 2017, which is hereby incorporated by reference in its entirety.

### FIELD OF THE INVENTION

The present disclosure relates to chewable gel products containing a gel carrier and an active pharmaceutical ingredient (API), where the chewable gel product is formulated to prevent or minimize the bitter taste characteristic of the API without utilizing taste-masking flavors or API-encapsulation.

### BACKGROUND

Many individuals find it difficult to take medications. For example, individuals may find it difficult to swallow tablets and capsules, particularly if they are large. Individuals may also avoid the unpleasant and unpalatable taste of medications, including both solid and liquid medicinal formulations. Most active pharmaceutical ingredients (APIs) are believed to possess an intrinsic bitter characteristic taste, leading to avoidance and difficulties in maintaining compliance with the regimen for administration of the medication. Avoidance of taking either prescribed or over-the-counter medications can lead to low levels of drug efficacy, resulting in prolonged or worsening symptoms and/or failure to treat or cure the indicated malady.

### BRIEF SUMMARY

There exists a clear need for alternative delivery vehicles for medications, such as active pharmaceutical ingredients, that are convenient, easy to self-administer, and have minimal unpleasant, bitter, or unpalatable tastes. Accordingly, the present disclosure relates, in part, to chewable gel products having an active pharmaceutical ingredient that has little to no bitter or unpalatable taste. In the extreme, if a pharmaceutical dosage form is too sweet, a patient may perceive it as not being an effective product if there is no pharmaceutical taste and could, consequently, be misinterpreted as a candy product. It is thus desirable to be able to control the intensity of the bitterness of the active pharmaceutical ingredient in the dosage form to produce medication delivery vehicle with little to no bitterness. The present disclosure addresses, at least in part, an unexpected and previously unrecognized problem relating to the use of active pharmaceutical ingredients in chewable gel products. In particular, it was found that pectin-based and gelatin-based products containing loratadine resulted in a surprisingly bitter and unpalatable taste profile (*See e.g.*, Example 1). Without wishing to be bound by theory, it is believed that this unexpected bitterness is due to the ionization and/or complexation of loratadine with the particular acid used for gelation of the gelatin-gelled or pectin-gelled product. As a consequence, this ionized or complexed active pharmaceutical ingredient-acid is able to bind and interact with the bitter sensing nerves in the oral cavity resulting in a bitter sensation. As such, the present disclosure relates, at least in part, to the surprising finding that specific acids in combination with specific pH ranges may be used in pectin-based or gelatin-based loratadine chewable gel products to overcome this previously unrecognized problem of pronounced bitterness from the loratadine. Advantageously, these particular combinations of acids and pH ranges led to non-bitter and palatable taste profiles of the resulting loratadine-containing chewable gels. Additionally, the present disclosure relates, at least in part, to the surprising finding that chewable gel products having one or more non-acid-activated gel carriers (e.g., non-pectin-based and non-gelatin based) are suitable for delivering loratadine without any bitter or unpalatable tastes, circumventing the unexpected issues of bitterness arising from pectin-based or gelatin-based, loratadine-containing chewable gel products.

Accordingly, certain aspects of the present disclosure relate to a chewable gel product, having a non-acid-activated gel carrier; and an active pharmaceutical ingredient. In certain embodiments, the chewable gel product has a taste acceptability index (TAI) of about 6 to about 10. In some embodiments, the non-acid-activated gel carrier includes carrageenan, agar, modified starch, acacia gum, alginic acid, locust bean gum or any combination thereof. In some embodiments, the non-acid-activated gel carrier includes carrageenan. In some embodiments, the non-acid-activated gel carrier includes a combination of carrageenan and pectin or a combination of carrageenan and gelatin. In some embodiments, the active pharmaceutical ingredient is selected from an anti-inflammatory, an antirheumatic, an antipyretic, an antiemetic, an analgesic, an antiepileptic, an antipsychotic, an antidepressant, a hypnotic, an anti-ulceric, a prokinetic, an anti-asthmatic, an antiparkinsonic, a cardiovascular, a vasodilator, a urologic, a diuretic, an erectile dysfunction medication, a hypolipidemic, an anti-diabetic, and an antihistaminic active ingredient. In some embodiments, the active pharmaceutical ingredient is selected from loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, ephedrine, ibuprofen, acetaminophen (paracetamol), ketoprofen, naproxen, piroxicam, meloxicam, leflunomide, ondansetron, granisetron, carbamazepine, lamotrigine, clozapine, olanzapine, risperidone, citalopram, paroxetine, sertraline, fluoxetine, fluvoxamine, zopiclon, zolpidem, cimetidine, ranitidine, omeprazole, metoclopramide, cisapride, domperidon, zafirlukast, montelukast, pramipexol, selegiline, doxazosin, terazosin, atenolol, bisoprolol, amlodipine, nifedipine, diltiazem, enalapril, captopril, ramipril, losartan, glyceroltrinitrate, alfuzosin, finasteride, pravastatin, atorvastatin, simvastatin, gemfibrozil, metformin, terfenadine, celecoxib, rifecoxib, rivastignine, astemizole, hydroxyzine, clemastine, local anesthestics, antiseptics, opioids, opioid derivatives, sildenafil, tadalafil, vardenafil, and a pharmaceutically acceptable salt, ester, hydrate or solvate thereof. In some embodiments, the active pharmaceutical ingredient is selected from loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, and a pharmaceutically acceptable salt, ester, hydrate, or solvate thereof. In some embodiments, the active pharmaceutical ingredient is loratadine, or a pharmaceutically acceptable salt, ester, hydrate, or solvate thereof. In some embodiments, the chewable gel product includes the active pharmaceutical ingredient in an amount that ranges from about 0.01% w/w to about 50% w/w. In some embodiments, the chewable gel product includes the non-acid-activated gel carrier in an amount that ranges from about 0.5% w/w to about 10% w/w.

Other aspects of the present disclosure relate to a chewable gel product, having a non-acid-activated gel carrier that includes carrageenan; and loratadine. In some embodiments, the chewable gel product has a taste acceptability index (TAI) of about 6 to about 10. In some embodiments, the chewable gel product includes the non-acid-activated gel carrier that includes carrageenan in an amount that ranges from about 0.5% w/w to about 5% w/w. In some embodiments, the chewable gel product includes the non-acid-activated gel carrier that includes carrageenan in an amount that ranges from about 0.75% w/w to about 5% w/w. In some embodiments, the chewable gel product includes the non-acid-activated gel carrier that includes carrageenan in an amount that ranges from about 1% w/w to about 3% w/w. In some embodiments, the chewable gel product includes loratadine in an amount that ranges from about 0.01% w/w to about 10% w/w. In some embodiments, the chewable gel product includes loratadine in an amount that ranges from about 0.05% w/w to about 5% w/w. In some embodiments, the chewable gel product includes loratadine in an amount that ranges from about 0.1% w/w to about 3% w/w.

Other aspects of the present disclosure relate to a chewable gel product, having a non-acid-activated gel carrier that includes carrageenan and pectin; and loratadine. In some embodiments, the chewable gel product has a taste acceptability index (TAI) of about 6 to about 10. In some embodiments, the chewable gel product includes the non-acid-activated gel carrier that includes carrageenan and pectin in an amount that ranges from about 0.5% w/w to about 10% w/w. In some embodiments, the chewable gel product includes the non-acid-activated gel carrier that includes carrageenan and pectin in an amount that ranges from about 0.75% w/w to about 5% w/w. In some embodiments, the chewable gel product includes the non-acid-activated gel carrier that includes carrageenan and pectin in an amount that ranges from about 1% w/w to about 3% w/w. In some embodiments, the chewable gel product includes loratadine in an amount that ranges from about 0.01% w/w to about 10% w/w. In some embodiments, the chewable gel product includes loratadine in an amount that ranges from about 0.05% w/w to about 5% w/w. In some embodiments, the chewable gel product includes loratadine in an amount that ranges from about 0.1% w/w to about 3% w/w.

In some embodiments that may be combined with any of the preceding embodiments, the chewable gel product has a taste acceptability index (TAI) of greater than about 6. In some embodiments that may be combined with any of the preceding embodiments, the chewable gel product has a taste acceptability index (TAI) of greater than about 7. In some embodiments that may be combined with any of the preceding embodiments, the chewable gel product has a taste acceptability index (TAI) of greater than about 8. In some embodiments that may be combined with any of the preceding embodiments, the chewable gel product has a taste acceptability index (TAI) of greater than about 9. In some embodiments that may be combined with any of the preceding embodiments, the chewable gel product has a taste acceptability index (TAI) of about 10. In some embodiments that may be combined with any of the preceding embodiments, the taste acceptability index (TAI) is measured prior to gelation. In some embodiments that may be combined with any of the preceding embodiments, the chewable gel product has a pH value that ranges from about 4 to about 8. In some embodiments that may be combined with any of the preceding embodiments, prior to gelation the product is in a liquid state and has a pH value that ranges from about 4 to about 8. In some embodiments that may be combined with any of the preceding embodiments, the chewable gel product has a pH value that ranges from about 3.9 to about 5. In some embodiments that may be combined with any of the preceding embodiments, prior to gelation the product is in a liquid state and has a pH value that ranges from about 3.9 to about 5. In some embodiments that may be combined with any of the preceding embodiments, the chewable gel product further includes one or more wetting agents. In some embodiments that may be combined with any of the preceding embodiments, the one or more wetting agents are selected from propylene glycol, propylene glycol alginate, glycerin, sorbitol, polyethylene glycol, maltitol, maltitol syrup, lecithin, and any combination thereof. In some embodiments that may be combined with any of the preceding embodiments, the chewable gel product includes the one or more wetting agents in an amount that ranges from about 0.1% w/w to about 20% w/w. In some embodiments that may be combined with any of the preceding embodiments, the chewable gel product further includes a pH-adjusting agent. In some embodiments that may be combined with any of the preceding embodiments, the pH-adjusting agent is one or more acids selected from citric acid, fumaric acid, malic acid, phosphoric acid, succinic acid, tartaric acid, maleic acid, acetic acid, hydrochloric acid, lactic acid, propionic acid, salts thereof, and derivatives thereof. In some embodiments that may be combined with any of the preceding embodiments, the pH-adjusting agent is malic acid, salts thereof, or derivatives thereof. In some embodiments that may be combined with any of the preceding embodiments, the pH-adjusting agent is phosphoric acid, salts thereof, or derivatives thereof. In some embodiments that may be combined with any of the preceding embodiments, the pH-adjusting agent is succinic acid, salts thereof, or derivatives thereof. In some embodiments that may be combined with any of the preceding embodiments, the chewable gel product further includes one or more base vehicles. In some embodiments that may be combined with any of the preceding embodiments, the one or more base vehicles are selected from maltitol, maltodextrin, maltitol syrup, corn syrup, xylitol, sucrose, dextrose, maltose, fructose, mannitol, erythritol, glycerin, isomalt, polydextrose, lactitol, lycasin, sorbitol, and any combination thereof. In some embodiments that may be combined with any of the preceding embodiments, the chewable gel product further includes one or more sweeteners. In some embodiments that may be combined with any of the preceding embodiments, the one or more sweeteners are selected from sucrose, aspartame, sucralose, saccharin, sodium saccharin, acesulfame potassium, stevia, sodium cyclamate, inulin, isomalt, maltose, neohesperidin dihydrochalcone, trehalose, thaumatin, sorbitol, maltitol, and any combination thereof. In some embodiments that may be combined with any of the preceding embodiments, the chewable gel product further includes a flavorant. In some embodiments that may be combined with any of the preceding embodiments, the flavorant includes one or more flavors selected from a mixed berry flavor, a mulberry flavor, a cherry flavor, a strawberry flavor, a citrus flavor, a lemon flavor, a lime flavor, an orange flavor, a grape flavor, a vanilla flavor, a chocolate flavor, a caramel flavor, a mint flavor, a spearmint flavor, a wintergreen flavor, and a menthol flavor. In some embodiments that may be combined with any of the preceding embodiments, the chewable gel product further includes one or more colorants. In some embodiments that may be combined with any of the preceding embodiments, the one or more colorants are selected from FD&C Red 40, D&C Reds 3, 22, 28, 33 and 36, FD&C Yellows 5 and 6, D&C Yellow 10, FD&C Blues 1 and 2, FD&C Green 3, red iron oxide, caramel, beta-carotene, carmine, and any combination thereof. In some embodiments that may be combined with any of the preceding embodiments, the chewable gel product has a chewable bite index (CBI) of about 2 to about 5. In some embodiments that may be combined with any of the preceding embodiments, the chewable gel product has a chewable bite index (CBI) of greater than about 3. In some embodiments that may be combined with any of the preceding embodiments, the chewable gel product has a chewable bite index (CBI) of greater than about 4. In some embodiments that may be combined with any of the preceding embodiments, the chewable gel product has a chewable bite index (CBI) of about 5.

Other aspects of the present disclosure relate to a chewable gel product, having an acid-activated gel carrier; an active pharmaceutical ingredient; and a taste-compatible pH adjusting agent. In some embodiments, the chewable gel product includes an amount of taste-compatible pH adjusting agent sufficient to yield a taste acceptability index (TAI) of about 6 to about 10. In some embodiments, the acid-activated gel carrier includes pectin or gelatin. In some embodiments, the acid-activated gel carrier includes pectin. In some embodiments, the acid-activated gel carrier includes gelatin. In some embodiments, the active pharmaceutical ingredient is selected from an anti-inflammatory, an antirheumatic, an antipyretic, an antiemetic, an analgesic, an antiepileptic, an antipsychotic, an antidepressant, a hypnotic, an anti-ulceric, a prokinetic, an anti-asthmatic, an antiparkinsonic, a cardiovascular, a vasodilator, a urologic, a diuretic, an erectile dysfunction medication, a hypolipidemic, an anti-diabetic, and an antihistaminic active ingredient. In some embodiments, the active pharmaceutical ingredient is selected from loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, ephedrine, ibuprofen, acetaminophen (paracetamol), ketoprofen, naproxen, piroxicam, meloxicam, leflunomide, ondansetron, granisetron, carbamazepine, lamotrigine, clozapine, olanzapine, risperidone, citalopram, paroxetine, sertraline, fluoxetine, fluvoxamine, zopiclon, zolpidem, cimetidine, ranitidine, omeprazole, metoclopramide, cisapride, domperidon, zafirlukast, montelukast, pramipexol, selegiline, doxazosin, terazosin, atenolol, bisoprolol, amlodipine, nifedipine, diltiazem, enalapril, captopril, ramipril, losartan, glyceroltrinitrate, alfuzosin, finasteride, pravastatin, atorvastatin, simvastatin, gemfibrozil, metformin, terfenadine, celecoxib, rifecoxib, rivastignine, astemizole, hydroxyzine, clemastine, local anesthestics, antiseptics, opioids, opioid derivatives, sildenafil, tadalafil, vardenafil, and a pharmaceutically acceptable salt, ester, hydrate or solvate thereof. In some embodiments, the active pharmaceutical ingredient is selected from loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, and a pharmaceutically acceptable salt, ester, hydrate, or solvate thereof. In some embodiments, the active pharmaceutical ingredient is loratadine, or a pharmaceutically acceptable salt, ester, hydrate, or solvate thereof. In some embodiments, the chewable gel product includes the active pharmaceutical ingredient in an amount that ranges from about 0.01% w/w to about 50% w/w. In some embodiments, the chewable gel product includes the acid-activated gel carrier in an amount that ranges from about 0.5% w/w to about 10% w/w. In some embodiments, the taste-compatible pH adjusting agent is one or more acids selected from fumaric acid, malic acid, phosphoric acid, succinic acid, tartaric acid, maleic acid, acetic acid, hydrochloric acid, lactic acid, propionic acid, salts thereof, and derivatives thereof. In some embodiments, the pH adjusting agent is malic acid, salts thereof, or derivatives thereof. In some embodiments, the pH adjusting agent is phosphoric acid, salts thereof, or derivatives thereof. In some embodiments, the pH adjusting agent is succinic acid, salts thereof, or derivatives thereof. In some embodiments, the chewable gel product includes the taste-compatible pH adjusting agent in an amount that yields a pH value that ranges from 2.5 to 4.0. In some embodiments, prior to gelation the product is in a liquid state and has a pH value that ranges from 2.5 to 4.0.

Other aspects of the present disclosure relate to a chewable gel product, having pectin; loratadine; and a taste-compatible acid. In some embodiments, the chewable gel product includes the acid in an amount sufficient to yield a taste acceptability index (TAI) of about 6 to about 10. In some embodiments, the chewable gel product includes the loratadine in an amount that ranges from about 0.01% w/w to about 10% w/w. In some embodiments, the chewable gel product includes the loratadine in an amount that ranges from about 0.05% w/w to about 5% w/w. In some embodiments, the chewable gel product includes the loratadine in an amount that ranges from about 0.1% w/w to about 3% w/w. In some embodiments, the chewable gel product includes the pectin in an amount that ranges from about 0.5% w/w to about 5% w/w. In some embodiments, the chewable gel product includes the pectin in an amount that ranges from about 0.75% w/w to about 4% w/w. In some embodiments, the chewable gel product includes the pectin in an amount that ranges from about 1% w/w to about 3% w/w. In some embodiments, the acid is one or more acids selected from fumaric acid, malic acid, phosphoric acid, succinic acid, tartaric acid, maleic acid, acetic acid, hydrochloric acid, lactic acid, propionic acid, salts thereof, and derivatives thereof. In some embodiments, the acid is malic acid, salts thereof, or derivatives thereof. In some embodiments, the acid is phosphoric acid, salts thereof, or derivatives thereof. In some embodiments, the acid is succinic acid, salts thereof, or derivatives thereof. In some embodiments, the chewable gel product includes the acid in an amount that yields a pH value that ranges from 2.5 to 4.0. In some embodiments, prior to gelation the product is in a liquid state and has a pH value that ranges from 2.5 to 4.0.

In some embodiments that may be combined with any of the preceding embodiments, the chewable gel product has a taste acceptability index (TAI) of greater than about 6. In some embodiments that may be combined with any of the preceding embodiments, the chewable gel product has a taste acceptability index (TAI) of greater than about 7. In some embodiments that may be combined with any of the preceding embodiments, the chewable gel product has a taste acceptability index (TAI) of greater than about 8. In some embodiments that may be combined with any of the preceding embodiments, the chewable gel product has a taste acceptability index (TAI) of greater than about 9. In some embodiments that may be combined with any of the preceding embodiments, the chewable gel product has a taste acceptability index (TAI) of about 10. In some embodiments that may be combined with any of the preceding embodiments, the taste acceptability index (TAI) is measured prior to gelation. In some embodiments that may be combined with any of the preceding embodiments, the chewable gel product further includes a wetting agent. In some embodiments that may be combined with any of the preceding embodiments, the wetting agent is selected from propylene glycol, propylene glycol alginate, glycerin, sorbitol, polyethylene glycol, maltitol syrup, lecithin, and any combination thereof. In some embodiments that may be combined with any of the preceding embodiments, the chewable gel product includes the wetting agent in an amount that ranges from about 0.1% w/w to about 20% w/w. In some embodiments that may be combined with any of the preceding embodiments, the chewable gel product further includes one or more base vehicles. In some embodiments that may be combined with any of the preceding embodiments, the one or more base vehicles are selected from maltitol, maltodextrin, maltitol syrup, corn syrup, xylitol, sucrose, dextrose, maltose, fructose, mannitol, erythritol, glycerin, isomalt, polydextrose, lactitol, lycasin, sorbitol, and any combination thereof. In some embodiments that may be combined with any of the preceding embodiments, the chewable gel product further includes one or more sweeteners. In some embodiments that may be combined with any of the preceding embodiments, the one or more sweeteners are selected from sucrose, aspartame, sucralose, saccharin, sodium saccharin, acesulfame potassium, stevia, sodium cyclamate, inulin, isomalt, maltose, neohesperidin dihydrochalcone, trehalose, thaumatin, sorbitol, maltitol, and any combination thereof. In some embodiments that may be combined with any of the preceding embodiments, the chewable gel product further includes a flavorant. In some embodiments that may be combined with any of the preceding embodiments, the flavorant includes one or more flavors selected from a mixed berry flavor, a mulberry flavor, a cherry flavor, a strawberry flavor, a citrus flavor, a lemon flavor, a lime flavor, an orange flavor, a grape flavor, a vanilla flavor, a chocolate flavor, a caramel flavor, a mint flavor, a spearmint flavor, a wintergreen flavor, and a menthol flavor. In some embodiments that may be combined with any of the preceding embodiments, the chewable gel product further includes one or more colorants. In some embodiments that may be combined with any of the preceding embodiments, the one or more colorants are selected from FD&C Red 40, D&C Reds 3, 22, 28, 33 and 36, FD&C Yellows 5 and 6, D&C Yellow 10, FD&C Blues 1 and 2, FD&C Green 3, red iron oxide, caramel, beta-carotene, carmine, and any combination thereof. In some embodiments that may be combined with any of the preceding embodiments, the chewable gel product has a chewable bite index (CBI) of about 2 to about 5. In some embodiments that may be combined with any of the preceding embodiments, the chewable gel product has a chewable bite index (CBI) of greater than about 3. In some embodiments that may be combined with any of the preceding embodiments, the chewable gel product has a chewable bite index (CBI) of greater than about 4. In some embodiments that may be combined with any of the preceding embodiments, the chewable gel product has a chewable bite index (CBI) of about 5.

Other aspects of the present disclosure relate to a chewable gel product, having carrageenan; and loratadine. In some embodiments, the chewable gel product has a taste acceptability index (TAI) of about 6 to about 10. In some embodiments, the chewable gel product includes the carrageenan in an amount that ranges from about 0.5% w/w to about 10% w/w. In some embodiments, the chewable gel product includes the carrageenan in an amount that ranges from about 0.75% w/w to about 5% w/w. In some embodiments, the chewable gel product includes the carrageenan in an amount that ranges from about 1% w/w to about 2% w/w. In some embodiments, the chewable gel product includes the carrageenan in an amount that ranges from about 1% w/w to about 2% w/w. In some embodiments, the chewable gel product includes about 1.8% w/w of carrageenan. In some embodiments, the chewable gel product includes loratadine in an amount that ranges from about 0.01% w/w to about 10% w/w. In some embodiments, the chewable gel product includes loratadine in an amount that ranges from about 0.05% w/w to about 5% w/w. In some embodiments, the chewable gel product includes loratadine in an amount that ranges from about 0.1% w/w to about 3% w/w. In some embodiments, the chewable gel product includes loratadine in an amount that ranges from about 0.1% w/w to about 1% w/w. In some embodiments, the chewable gel product includes about 0.5% w/w of loratadine. In some embodiments, the gel product further includes sorbitol. In some embodiments, the chewable gel product contains sorbitol an amount that ranges from about 10% w/w to about 60% w/w. In some embodiments, the chewable gel product contains sorbitol in an amount that ranges from about 45% w/w to about 60% w/w. In some embodiments, the chewable gel product contains about 51 % w/w of sorbitol. In some embodiments, the chewable gel product contains about 58 % w/w of sorbitol. In some embodiments, the chewable gel product further includes one or more of maltitol and propylene glycol. In some embodiments, the chewable gel product further includes maltitol and propylene glycol. In some embodiments, the chewable gel product contains each of the maltitol and propylene glycol in an amount that ranges from about 1% w/w to about 25% w/w. In some embodiments, the chewable gel product contains maltitol in an amount that ranges from about 14% w/w to about 22% w/w. In some embodiments, the chewable gel product contains about 14.5 % w/w of maltitol. In some embodiments, the chewable gel product contains about 22 % w/w of maltitol. In some embodiments, the chewable gel product contains propylene glycol in an amount that ranges from about 1% w/w to about 10% w/w. In some embodiments, the chewable gel product contains about 3 % w/w of propylene glycol. In some embodiments, the chewable gel product further includes one or more sweeteners. In some embodiments, the one or more sweeteners are selected from aspartame, sucralose, and any combination thereof. In some embodiments, the one or more sweeteners are aspartame and sucralose. In some embodiments, the chewable gel product contains each of the one or more sweeteners in an amount that ranges from about 0.01% w/w to about 5% w/w. In some embodiments, the chewable gel product contains each of the one or more sweeteners in an amount that ranges from about 0.10% w/w to about 1% w/w. In some embodiments, the chewable gel product contains aspartame in an amount that ranges from about 0.10% w/w to about 0.50% w/w. In some embodiments, the chewable gel product contains about 0.20 % w/w of aspartame. In some embodiments, the chewable gel product contains sucralose in an amount that ranges from about 0.10% w/w to about 0.50% w/w. In some embodiments, the chewable gel product contains about 0.20 % w/w of sucralose. In some embodiments, the chewable gel product further includes water. In some embodiments, the chewable gel product contains water in an amount that ranges from about 5% w/w to about 25% w/w. In some embodiments, the chewable gel product contains water in an amount that ranges from about 15% w/w to about 25% w/w. In some embodiments, the chewable gel product contains about 21 % w/w of water. In some embodiments, the chewable gel product further includes one or more flavorants. In some embodiments, the chewable gel product further includes a flavorant. In some embodiments, the chewable gel product contains the flavorant in an amount that ranges from about 0.05% w/w to about 2% w/w. In some embodiments, the chewable gel product contains the flavorant in an amount that ranges from about 0.10% w/w to about 1% w/w. In some embodiments, the chewable gel product contains the flavorant in an amount that ranges from about 0.10% w/w to about 0.5% w/w. In some embodiments, the chewable gel product contains about 0.40 % w/w of flavorant. In some embodiments, the flavorant is a mulberry flavor. In some embodiments, the chewable gel product further includes one or more colorants. In some embodiments, the chewable gel product further includes a colorant. In some embodiments, the chewable gel product contains the colorant in an amount that ranges from about 0.001% w/w to about 0.3% w/w. In some embodiments, the chewable gel product contains the colorant in an amount that ranges from about 0.010% w/w to about 0.10% w/w. In some embodiments, the chewable gel product contains about 0.015 % w/w of colorant. In some embodiments, the colorant is FD&C Red No. 40. In some embodiments, the chewable gel product further includes a coating. In some embodiments, the coating includes one or more of MCT coconut oil and carnauba wax. In some embodiments, the coating includes MCT coconut oil and carnauba wax. In some embodiments, the chewable gel product further includes a source of cations. In some embodiments, the source of cations is potassium citrate. In some embodiments, the chewable gel product contains potassium citrate in an amount that ranges from about 0.10% w/w to about 1% w/w. In some embodiments, the chewable gel product contains about 0.20 % w/w of potassium citrate.

Other aspects of the present disclosure relate to a method for producing a chewable gel product, by: a, formulating a liquid composition having a gel carrier and an active pharmaceutical ingredient; b. adjusting the pH of the liquid composition to a pH that ranges from about 2.5 to about 8; and c. gelating the liquid composition at a temperature sufficient to yield a chewable gel product. In some embodiments, the gel carrier is a non-acid-activated gel carrier. In some embodiments, the non-acid-activated gel carrier includes carrageenan, agar, modified starch, acacia gum, alginic acid, locust bean gum or any combination thereof. In some embodiments, the non-acid-activated gel carrier includes carrageenan. In some embodiments, the non-acid-activated gel carrier includes a combination of carrageenan and pectin or a combination of carrageenan and gelatin. In some embodiments, the non-acid-activated gel carrier includes carrageenan in an amount that ranges from about 0.5% w/w to about 5% w/w. In some embodiments, the non-acid-activated gel carrier includes carrageenan in an amount that ranges from about 0.75% w/w to about 5% w/w. In some embodiments, the non-acid-activated gel carrier includes carrageenan in an amount that ranges from about 1% w/w to about 3% w/w. In some embodiments, the non-acid-activated gel carrier includes carrageenan and pectin in an amount that ranges from about 0.5% w/w to about 10% w/w. In some embodiments, the non-acid-activated gel carrier includes carrageenan and pectin in an amount that ranges from about 0.75% w/w to about 5% w/w. In some embodiments, the non-acid-activated gel carrier includes carrageenan and pectin in an amount that ranges from about 1% w/w to about 3% w/w. In some embodiments, the temperature sufficient to yield a chewable gel product includes heating the liquid composition having a non-acid-activated gel carrier and an active pharmaceutical ingredient to a temperature that ranges from about 100°C to about 125°C and then allowing the heated composition to gelate upon cooling. In some embodiments, the pH of the liquid composition having a non-acid-activated gel carrier is adjusted to a pH that ranges from about 3.9 to about 5. In some embodiments, the gel carrier is an acid-activated gel carrier. In some embodiments, the acid-activated gel carrier includes pectin or gelatin. In some embodiments, the acid-activated gel carrier includes pectin. In some embodiments, the acid-activated gel carrier includes pectin in an amount that ranges from about 0.5% w/w to about 5% w/w. In some embodiments, the acid-activated gel carrier includes pectin in an amount that ranges from about 0.75% w/w to about 4% w/w. In some embodiments, the acid-activated gel carrier includes pectin in an amount that ranges from about 1% w/w to about 3% w/w. In some embodiments, the acid-activated gel carrier includes gelatin. In some embodiments, the temperature sufficient to yield a chewable gel product includes heating the liquid composition having an acid-activated gel carrier and an active pharmaceutical ingredient to a temperature that ranges from about 45°C to about 90°C and then allowing the heated composition to gelate upon cooling. In some embodiments, the pH of the liquid composition having an acid-activated gel carrier is adjusted to a pH that ranges from about 4 to about 8. In some embodiments, the method further includes adding a taste-compatible acid to the liquid composition. In some embodiments, the taste-compatible acid is one or more acids selected from citric acid, fumaric acid, malic acid, phosphoric acid, succinic acid, tartaric acid, maleic acid, acetic acid, hydrochloric acid, lactic acid, propionic acid, salts thereof, and derivatives thereof. In some embodiments, the taste-compatible acid is malic acid, salts thereof, or derivatives thereof. In some embodiments, the taste-compatible acid is phosphoric acid, salts thereof, or derivatives thereof. In some embodiments, the taste-compatible acid is succinic acid, salts thereof, or derivatives thereof. In some embodiments, the taste-compatible acid is added in an amount that yields a pH value that ranges from 2.5 to 4.0. In some embodiments, the active pharmaceutical ingredient is selected from an anti-inflammatory, an antirheumatic, an antipyretic, an antiemetic, an analgesic, an antiepileptic, an antipsychotic, an antidepressant, a hypnotic, an anti-ulceric, a prokinetic, an anti-asthmatic, an antiparkinsonic, a cardiovascular, a vasodilator, a urologic, a diuretic, an erectile dysfunction medication, a hypolipidemic, an anti-diabetic, and an antihistaminic active ingredient. In some embodiments, the active pharmaceutical ingredient is selected from loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, ephedrine, ibuprofen, acetaminophen (paracetamol), ketoprofen, naproxen, piroxicam, meloxicam, leflunomide, ondansetron, granisetron, carbamazepine, lamotrigine, clozapine, olanzapine, risperidone, citalopram, paroxetine, sertraline, fluoxetine, fluvoxamine, zopiclon, zolpidem, cimetidine, ranitidine, omeprazole, metoclopramide, cisapride, domperidon, zafirlukast, montelukast, pramipexol, selegiline, doxazosin, terazosin, atenolol, bisoprolol, amlodipine, nifedipine, diltiazem, enalapril, captopril, ramipril, losartan, glyceroltrinitrate, alfuzosin, finasteride, pravastatin, atorvastatin, simvastatin, gemfibrozil, metformin, terfenadine, celecoxib, rifecoxib, rivastignine, astemizole, hydroxyzine, clemastine, local anesthestics, antiseptics, opioids, opioid derivatives, sildenafil, tadalafil, vardenafil, and a pharmaceutically acceptable salt, ester, hydrate or solvate thereof. In some embodiments, the active pharmaceutical ingredient is selected from loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, and a pharmaceutically acceptable salt, ester, hydrate, or solvate thereof. In some embodiments, the active pharmaceutical ingredient is loratadine, or a pharmaceutically acceptable salt, ester, hydrate, or solvate thereof. In some embodiments, the active pharmaceutical ingredient is present in an amount that ranges from about 0.01% w/w to about 50% w/w. In some embodiments, the active pharmaceutical ingredient includes loratadine in an amount that ranges from about 0.01% w/w to about 10% w/w. In some embodiments, the active pharmaceutical ingredient includes loratadine in an amount that ranges from about 0.05% w/w to about 5% w/w. In some embodiments, the active pharmaceutical ingredient includes loratadine in an amount that ranges from about 0.1% w/w to about 3% w/w. In certain embodiments, the chewable gel product has a taste acceptability index (TAI) of about 6 to about 10. In some embodiments, the chewable gel product further includes a wetting agent. In some embodiments, the wetting agent is selected from propylene glycol, propylene glycol alginate, glycerin, sorbitol, polyethylene glycol, maltitol syrup, lecithin, and any combination thereof. In some embodiments, the chewable gel product includes the wetting agent in an amount that ranges from about 0.1% w/w to about 20% w/w. In some embodiments, the chewable gel product further includes a pH-adjusting agent. In some embodiments, the pH-adjusting agent is one or more acids selected from citric acid, fumaric acid, malic acid, phosphoric acid, succinic acid, tartaric acid, maleic acid, acetic acid, hydrochloric acid, lactic acid, propionic acid, salts thereof, and derivatives thereof. In some embodiments, the pH-adjusting agent is malic acid, salts thereof, or derivatives thereof. In some embodiments, the pH-adjusting agent is phosphoric acid, salts thereof, or derivatives thereof. In some embodiments, the pH-adjusting agent is succinic acid, salts thereof, or derivatives thereof. In some embodiments, the chewable gel product further includes one or more base vehicles. In some embodiments, the one or more base vehicles are selected from maltitol, maltodextrin, maltitol syrup, corn syrup, xylitol, sucrose, dextrose, maltose, fructose, mannitol, erythritol, glycerin, isomalt, polydextrose, lactitol, lycasin, sorbitol, and any combination thereof. In some embodiments, the chewable gel product further includes one or more sweeteners. In some embodiments, the one or more sweeteners are selected from sucrose, aspartame, sucralose, saccharin, sodium saccharin, acesulfame potassium, stevia, sodium cyclamate, inulin, isomalt, maltose, neohesperidin dihydrochalcone, trehalose, thaumatin, sorbitol, maltitol, and any combination thereof. In some embodiments, the chewable gel product further includes a flavorant. In some embodiments, the flavorant includes one or more flavors selected from a mixed berry flavor, a mulberry flavor, a cherry flavor, a strawberry flavor, a citrus flavor, a lemon flavor, a lime flavor, an orange flavor, a grape flavor, a vanilla flavor, a chocolate flavor, a caramel flavor, a mint flavor, a spearmint flavor, a wintergreen flavor, and a menthol flavor. In some embodiments, the chewable gel product further includes one or more colorants. In some embodiments, the one or more colorants are selected from FD&C Red 40, D&C Reds 3, 22, 28, 33 and 36, FD&C Yellows 5 and 6, D&C Yellow 10, FD&C Blues 1 and 2, FD&C Green 3, red iron oxide, caramel, beta-carotene, carmine, and any combination thereof. In some embodiments, the chewable gel product has a chewable bite index (CBI) of about 2 to about 5.

It is to be understood that one, some, or all of the properties of the various embodiments described herein may be combined to form other embodiments of the present invention. These and other aspects of the invention will become apparent to one of skill in the art.

### BRIEF DESCRIPTION OF THE DRAWINGS

**FIG. 1** shows results of the effect of pH on the taste acceptability index (TAI) for loratadine-containing formulations using the indicated acids.
**FIG. 2** shows results of the effect of sweetener on the taste acceptability index of loratadine-containing formulations. The taste acceptability index for each of the indicated formulations was independently assessed by three tasters: PDR, RB, and RS.
**FIG. 3** shows the taste acceptability index of products having various active pharmaceutical ingredients (APIs).

### DETAILED DESCRIPTION

The following description sets forth exemplary methods, parameters, and the like. It should be recognized, however, that such description is not intended as a limitation on the scope of the present disclosure but is instead provided as a description of exemplary embodiments.

### Compositions

Certain aspects of the present disclosure relate to chewable gel products containing one or more gel carriers and one or more active pharmaceutical ingredients (API). In some embodiments, chewable gel products of the present disclosure may be used to deliver drug substances, such as APIs, via the oral route. The chewable gel products may include, without limitation, one or more gel carriers, one or more pH adjusting agents, one or more wetting agents, one or more base vehicles, one or more sweeteners, one or more flavorants, one or more colorants, and/or water. In some embodiments, the chewable gel products may maintain their molded shape, may be elastic, and/or may yield to mastication. In some embodiments, the chewable gel products can be chewed before swallowing. In some embodiments, the chewable gel products may also be referred to as gummies, soft chews, or jellies.

In some embodiments, the chewable gel products are formulated such that a controlled level of bitterness where little to no bitter or unpalatable taste profile from the API is detectable in the final formulation. In some embodiments, the chewable gel product has a taste acceptability index (TAI) of about 6 to about 10.

In some embodiments, the chewable gel product further contains one or more pH adjusting agents. In some embodiments, the chewable gel product further contains one or more wetting agents. In some embodiments, the chewable gel product further contains one or more base vehicles. In some embodiments, the chewable gel product further contains one or more sweeteners. In some embodiments, the chewable gel product further contains one or more flavorants. In some embodiments, the chewable gel product further contains one or more colorants.

In some embodiments, the chewable gel product contains one or more gel carriers, one or more APIs, and one or more (*e.g*., one or more, two or more, three or more, four or more, five or more, or all six) of one or more pH adjusting agents, one or more wetting agents, one or more base vehicles, one or more sweeteners, one or more flavorants, or one or more colorants. In some embodiments, the chewable gel product contains one or more gel carriers, one or more APIs, and one or more (*e.g*., one or more, two or more, three or more, four or more, or all five) one or more wetting agents, one or more base vehicles, one or more sweeteners, one or more flavorants, or one or more colorants.

In some embodiments, chewable gel products of the present disclosure are formulated to provide a pleasing mouthfeel. For example, the chewable gel product may be formulated to provide a soft, clean bite. In some embodiments, the chewable gel product is formulated to provide a firm, clean bite. In some embodiments, the chewable gel product is formulated to provide a texture similar to gummy candies (*e.g*., gummy bears) or gummy multivitamins (*e.g*., One A Day^{®} VitaCraves^{®}). In some embodiments, the chewable gel product has a chewable bite index (CBI) of about 2 to about 5.

### Gel Carriers

In some aspects, the present disclosure relates to chewable gel products containing one or more (*e.g.*, one or more, two or more, three or more, four or more, five or more, *etc.*) gel carriers. In some embodiments, chewable gel products of the present disclosure may be formed using one or more gelling agents as gel carriers. In some embodiments, the one or more gelling agents present in the final chewable gel product formulation are the one or more gel carriers. In some embodiments, an active pharmaceutical ingredient (API) of the present disclosure may be contained within the one or more gel carriers in the chewable gel product.

In some embodiments, gel carriers of the present disclosure are one or more non-acid-activated gel carriers that are activated or otherwise induced to form a gel in the absence of an acid (*e.g*., carrageenan). Such non-acid-activated gel carriers may be activated or otherwise induced to form a gel via some other means (*e.g*., by temperature or ionization). Suitable non-acid-activated gel carriers may include, for example, carrageenan, low-methoxyl pectin, agar, modified starch, acacia gum, alginic acid, locust bean gum, derivatives thereof, and any combination thereof. Carrageenan refers to a group of sulfated galactans that can be extracted from red seaweeds. This group includes high molecular weight polysaccharides with repeating galactose units and 3,6-anhydrogalactose (both sulphated and non-sulphated). The units are joined by alternating alpha-1,3 and beta-1,4 glycosidic linkages. Carrageenans can be distinguished in part by the degree and position of sulfation. Most sugar units have one or two sulfate groups esterified to a hydroxyl group on carbons 2 or 6. Suitable carrageenans may include, without limitation, kappa carrageenan, lambda carrageenan, iota carrageenan, kappa II carrageenan, and mixtures thereof. In some embodiments, cations may be used to in the carrageenan gelation process. Suitable cations may include, without limitation, sodium (Na⁺), potassium (K⁺), lithium (Li⁺), calcium (Ca²⁺), and magnesium (Mg²⁺). In some embodiments, kappa carrageenans can produce strong rigid gels. In some embodiments, iota carrageenans are elastic and deformable. In some embodiments, kappa II carrageenan, which is a copolymer of kappa and iota carrageenans, can form weak gels. In some embodiments, lambda carrageenan does not form gels in water, but can be useful in blends, for example, to modify the mechanical properties of the resulting gel. In some embodiments, low-methoxyl pectin does not dependent on the presence of an acid and can gel at pH values between about 2.6 and about 7. Cations may be used in the low-methoxyl pectin gelation process. Suitable cations include, without limitation, sodium (Na⁺), potassium (K), lithium (Li⁺), calcium (Ca²⁺), and magnesium (Mg²⁺). In some embodiments, the chewable gel product contains cations (*e.g*., potassium citrate) in an amount that ranges from about 0.10% w/w to about 1% w/w. In some embodiments, the chewable gel product contains about 0.10% w/w of cations, about 0.20% w/w of cations, about 0.30% w/w of cations, about 0.40% w/w of cations, about 0.50% w/w of cations, about 0.60% w/w of cations, about 0.70% w/w of cations, about 0.80% w/w of cations, about 0.90% w/w of cations, or about 1% w/w of cations. It should be noted that the % w/w cation values described herein may vary by ±0.02. For example 0.1% w/w cation could vary from0.098% w/w cation to 0.12% w/w cation. In some embodiments, the non-acid-activated gel carriers contain carrageenans capable of forming ionotropic gels with multivalent ions (*e.g*., iota-carrageenan). In some embodiments, the non-acid-activated gel carrier contains carrageenan. In some embodiments, the non-acid-activated gel carrier contains low-methoxyl pectin.

Alternatively, gel carriers of the present disclosure are one or more acid-activated gel carriers that are activated or otherwise induced to form a gel in the presence of an acid (*e.g*., pectin and gelatin). Suitable acid-activated gel carriers may include, for example, pectin, gelatin, and any combinations thereof. Pectin is a heterogeneous complex polysaccharide found in higher plants, however commercially it is usually derived from citrus peel or apple pomace. Structurally, pectin is composed of linear segments of 1,4-linked α-D galactopyranosyluronic acid units in which some of the carboxyl groups are esterified with methanol. In addition, the hydroxyl groups of certain sources of pectin are also esterified with acetic acid. Pectin may also be treated with ammonia to form amidated pectin. Pectins can be classified as either high-methoxyl pectin or low-methoxyl pectin. In some embodiments, high-methoxyl pectin utilizes an acid for gelation and can gel at pH values between 2.8 and 3.6. In some embodiments, the acid-activated gel carrier contains pectin. In some embodiments, the acid-activated gel carrier contains high-methoxyl pectin. In some embodiments, the acid-activated gel carrier contains gelatin.

In some embodiments, the one or more gel carriers are a combination of one or more non-acid-activated gel carriers and one or more acid-activated gel carriers (*e.g*., carrageenan + pectin, carrageenan + gelatin, carrageenan + pectin + gelatin, *etc.*)*.*

In some embodiments, the chewable gel product contains the one or more gel carriers in an amount that ranges from about 0.5% w/w to about 10% w/w. In some embodiments, the chewable gel product contains the one or more gel carriers in an amount that is less than about 10% w/w, less than about 9.5% w/w, less than about 9% w/w, less than about 8.5% w/w, less than about 8% w/w, less than about 7.5% w/w, less than about 7% w/w, less than about 6.5% w/w, less than about 6% w/w, less than about 5.5% w/w, less than about 5% w/w, less than about 4.5% w/w, less than about 4% w/w, less than about 3.75% w/w, less than about 3.5% w/w, less than about 3.25% w/w, less than about 3% w/w, less than about 2. 75% w/w, less than about 2.5% w/w, less than about 2.25% w/w, less than about 2% w/w, less than about 1.75% w/w, less than about 1.5% w/w, less than about 1.25% w/w, less than about 1% w/w, or less than about 0.75% w/w. In some embodiments, the chewable gel product contains the one or more gel carriers in an amount that is greater than about 0.5% w/w, greater than about 0.75% w/w, greater than about 1% w/w, greater than about 1.25% w/w, greater than about 1.5% w/w, greater than about 1.75% w/w, greater than about 2% w/w, greater than about 2.25% w/w, greater than about 2.5% w/w, greater than about 2.75% w/w, greater than about 3% w/w, greater than about 3.25% w/w, greater than about 3.5% w/w, greater than about 3.75% w/w, greater than about 4% w/w, greater than about 4.5% w/w, greater than about 5% w/w, greater than about 5.5% w/w, greater than about 6% w/w, greater than about 6.5% w/w, greater than about 7% w/w, greater than about 7.5% w/w, greater than about 8% w/w, greater than about 8.5% w/w, or greater than about 9% w/w. In some embodiments, the chewable gel product contains the one or more gel carriers in an amount that has a range of % w/w having an upper limit of about 10% w/w, 9.5% w/w, 9% w/w, 8.5% w/w, 8% w/w, 7.5% w/w, 7% w/w, 6.5% w/w, 6% w/w, 6.5% w/w, 5% w/w, 4.5% w/w, 4% w/w, 3.75% w/w, 3.5% w/w, 3.25% w/w, 3% w/w, 2.75% w/w, 2.5% w/w, 2.25% w/w, 2% w/w, 1.75% w/w, 1.5% w/w, 1.25% w/w, 1% w/w, or 0.75% w/w, and an independently selected lower limit of about 0.5% w/w, 0.75% w/w, 1% w/w, 1.25% w/w, 1.5% w/w, 1.75% w/w, 1.8% w/w, 2% w/w, 2.25% w/w, 2.5% w/w, 2.75% w/w, 3% w/w, 3.25% w/w, 3.5% w/w, 3.75% w/w, 4% w/w, 4.5% w/w, 5.0%, 5.5% w/w, 6.0%, 6.5% w/w, 7.0%, 7.5% w/w, 8.0%, 8.5% w/w, or 9% w/w, where the lower limit is less than the upper limit. In some embodiments, the chewable gel product contains the one or more gel carriers in an amount of about 0.5% w/w, about 0.75% w/w, about 1% w/w, about 1.25% w/w, about 1.5% w/w, about 1.75% w/w, about 2% w/w, about 2.25% w/w, about 2.5% w/w, about 2.75% w/w, about 3% w/w, about 3.25% w/w, about 3.5% w/w, about 3.75% w/w, about 4% w/w, about 4.5% w/w, about 5% w/w, about 5.5% w/w, about 6.0%, about 6.5% w/w, about 7.0% w/w, about 7.5% w/w, about 8.0%, about 8.5% w/w, about 9.0%, about 9.5% w/w, or about 10% w/w. In some embodiments, the chewable gel product contains the one or more gel carriers in an amount of about 1% w/w. In some embodiments, the chewable gel product contains the one or more gel carriers in an amount of about 2% w/w. In some embodiments, the chewable gel product contains the one or more gel carriers in an amount of about 5% w/w. In some embodiments, the chewable gel product contains the one or more gel carriers in an amount of about 6% w/w. In some embodiments, the chewable gel product contains the one or more gel carriers in an amount of about 7% w/w. It should be noted that the % w/w gel carrier values described herein may vary by ±0.2. For example 2% w/w gel carrier could vary from 1.8% w/w gel carrier to 2.2% w/w gel carrier.

### Active Pharmaceutical Ingredients

In some aspects, the present disclosure relates to chewable gel products containing one or more (*e.g.*, one or more, two or more, three or more, four or more, five or more, *etc.*) active pharmaceutical ingredients (APIs). In some embodiments, the one or more APIs are one or more of an anti-inflammatory, an antirheumatic, an antipyretic, an antiemetic, an analgesic, an antiepileptic, an antipsychotic, an antidepressant, a hypnotic, an anti-ulceric, a prokinetic, an anti-asthmatic, an antiparkinsonic, a cardiovascular, a vasodilator, a urologic, a diuretic, an erectile dysfunction medication, a hypolipidemic, an anti-diabetic, an antihistaminic active ingredient, and any combinations thereof. Examples of suitable active pharmaceutical ingredients may include, without limitation, loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, ephedrine, ibuprofen, acetaminophen (paracetamol), ketoprofen, naproxen, piroxicam, meloxicam, leflunomide, ondansetron, granisetron, carbamazepine, lamotrigine, clozapine, olanzapine, risperidone, citalopram, paroxetine, sertraline, fluoxetine, fluvoxamine, zopiclon, zolpidem, cimetidine, ranitidine, omeprazole, metoclopramide, cisapride, domperidon, zafirlukast, montelukast, pramipexol, selegiline, doxazosin, terazosin, atenolol, bisoprolol, amlodipine, nifedipine, diltiazem, enalapril, captopril, ramipril, losartan, glyceroltrinitrate, alfuzosin, finasteride, pravastatin, atorvastatin, simvastatin, gemfibrozil, metformin, terfenadine, celecoxib, rifecoxib, rivastignine, astemizole, hydroxyzine, clemastine, local anesthestics, antiseptics, opioids (*e.g.*, morphine, *etc.*), opioid derivatives (*e.g.*, morphine derivatives), sildenafil, tadalafil, vardenafil, as well as any pharmaceutically acceptable salts, esters, hydrates or solvates thereof. In some embodiments, the API is one or more of loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, and a pharmaceutically acceptable salt, ester, hydrate, or solvate thereof. In some embodiments, the API is loratadine, or a pharmaceutically acceptable salt, ester, hydrate, or solvate thereof. In some embodiments, the API is diphenhydramine, or a pharmaceutically acceptable salt, ester, hydrate, or solvate thereof (*e.g.*, diphenhydramine HCl, diphenhydramine citrate, *etc.*)*.* In some embodiments, the API is desloratadine, or a pharmaceutically acceptable salt, ester, hydrate, or solvate thereof. In some embodiments, the API is phenylephrine, or a pharmaceutically acceptable salt, ester, hydrate, or solvate thereof (*e.g*., phenylephrine HCl, phenylephrine bitartrate, *etc.*)*.* In some embodiments, the API is chlorpheniramine, or a pharmaceutically acceptable salt, ester, hydrate, or solvate thereof (*e.g*., chlorpheniramine maleate, *etc.*)*.* In some embodiments, the API is dextromethorphan, or a pharmaceutically acceptable salt, ester, hydrate, or solvate thereof (*e.g*., dextromethorphan HBr, *etc.*)*.* In some embodiments, the API is doxylamine, or a pharmaceutically acceptable salt, ester, hydrate, or solvate thereof (*e.g*., doxylamine succinate, *etc.*)*.* In some embodiments, the API is guaifenesin, or a pharmaceutically acceptable salt, ester, hydrate, or solvate thereof. In some embodiments, the API is fexofenadine, or a pharmaceutically acceptable salt, ester, hydrate, or solvate thereof (*e.g*., fexofenadine HCl, *etc.*)*.* In some embodiments, the API is docusate, or a pharmaceutically acceptable salt, ester, hydrate, or solvate thereof (*e.g*., docusate sodium, *etc.*)*.* In some embodiments, the API is pseudoephedrine, or a pharmaceutically acceptable salt, ester, hydrate, or solvate thereof (*e.g*., pseudoephedrine HCl, pseudoephedrine sulfate, *etc.*)*.* In some embodiments, the API is cetirizine, or a pharmaceutically acceptable salt, ester, hydrate, or solvate thereof (*e.g*., cetirizine HCl, *etc.*)*.* In some embodiments, the API is triprolidine, or a pharmaceutically acceptable salt, ester, hydrate, or solvate thereof (*e.g*., triprolidine HCl, *etc.*)*.* In some embodiments, the API is brompheniramine, or a pharmaceutically acceptable salt, ester, hydrate, or solvate thereof (*e.g*., brompheniramine maleate, *etc.*)*.*

In some embodiments, the chewable gel product contains one or more APIs in a therapeutically effective amount. In some embodiments, the chewable gel product contains one or more APIs in an amount that ranges from about 0.01% w/w to about 50% w/w. As used herein, the term "*percent weight*/*weight (% w*/*w*)" refers to the weight of the indicated component as a percentage of the total weight of the final formulation of an individual chewable gel product of the present disclosure. In some embodiments, the chewable gel product contains one or more APIs in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains one or more APIs in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains one or more APIs in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, 0.5% w/w, or 0.1% w/w, and an independently selected lower limit of about 0.01% w/w, 0.05% w/w, 0.1% w/w/, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains one or more APIs in an amount of at about 0.01% w/w, about 0.05% w/w, about 0.1% w/w, about 0.25% w/w, about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 15% w/w, about 20% w/w, about 25% w/w, about 30% w/w, about 35% w/w, about 40% w/w, about 45% w/w, or about 50% w/w. It should be noted that the % w/w API values described herein may vary by ±0.2. For example 1% w/w API could vary from 0.98% w/w API to 1.2% w/w API.

In some embodiments, the chewable gel product contains one or more APIs in an amount that ranges from about 0.1 mg/ unit dose to about 500 mg/ unit dose. As used herein, the term "*mg*/ *unit dose*" refers to the total weight of the indicated component as present in the final formulation of an individual chewable gel product of the present disclosure. In some embodiments, the chewable gel product contains one or more APIs in an amount that is less than about 500 mg/ unit dose, less than about 450 mg/ unit dose, less than about 400 mg/ unit dose, less than about 350 mg/ unit dose, less than about 300 mg/ unit dose, less than about 250 mg/ unit dose, less than about 200 mg/ unit dose, less than about 150 mg/ unit dose, less than about 100 mg/ unit dose, less than about 75 mg/ unit dose, less than about 50 mg/ unit dose, less than about 25 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, less than about 2 mg/ unit dose, less than about 1 mg/ unit dose, less than about 0.75 mg/ unit dose, or less than about 0.25 mg/ unit dose. In some embodiments, the chewable gel product contains one or more APIs in an amount that is greater than about 0.1 mg/ unit dose, greater than about 0.25 mg/ unit dose, greater than about 0.75 mg/ unit dose, greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 25 mg/ unit dose, greater than about 50 mg/ unit dose, greater than about 75 mg/ unit dose, greater than about 100 mg/ unit dose, greater than about 150 mg/ unit dose, greater than about 200 mg/ unit dose, greater than about 250 mg/ unit dose, greater than about 300 mg/ unit dose, greater than about 350 mg/ unit dose, greater than about 400 mg/ unit dose, or greater than about 450 mg/ unit dose. In some embodiments, the chewable gel product contains one or more APIs in an amount that has a range of mg/ unit dose having an upper limit of about 500 mg/ unit dose, 450 mg/ unit dose, 400 mg/ unit dose, 350 mg/ unit dose, 300 mg/ unit dose, 250 mg/ unit dose, 200 mg/ unit dose, 150 mg/ unit dose, 100 mg/ unit dose, 75 mg/ unit dose, 50 mg/ unit dose, 25 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, 2 mg/ unit dose, 1 mg/ unit dose, 0.75 mg/ unit dose, or 0.25 mg/ unit dose, and an independently selected lower limit of about 0.1 mg/ unit dose, 0.25 mg/ unit dose, 0.75 mg/ unit dose, 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose,10 mg/ unit dose, 25 mg/ unit dose, 50 mg/ unit dose, 75 mg/ unit dose, 100 mg/ unit dose, 150 mg/ unit dose, 200 mg/ unit dose, 250 mg/ unit dose, 300 mg/ unit dose, 350 mg/ unit dose, 400 mg/ unit dose, or 450 mg/ unit dose, where the lower limit is less than the upper limit. In some embodiments, the chewable gel product contains one or more APIs in an amount of about 0.1 mg/ unit dose, about 0.25 mg/ unit dose, about 0.75 mg/ unit dose, about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 25 mg/ unit dose, about 50 mg/ unit dose, about 75 mg/ unit dose, about 100 mg/ unit dose, about 150 mg/ unit dose, about 200 mg/ unit dose, about 250 mg/ unit dose, about 300 mg/ unit dose, about 350 mg/ unit dose, about 400 mg/ unit dose, about 450 mg/ unit dose, or about 500 mg/ unit dose.

In some embodiments, the chewable gel product contains loratadine, or a pharmaceutically acceptable salt, ester, hydrate, or solvate thereof, in an amount that ranges from about0.01% w/w to about 50% w/w. In some embodiments, the chewable gel product contains loratadine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains loratadine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains loratadine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains loratadine, or a pharmaceutically acceptable salt, ester, hydrate, or solvate thereof, in an amount of at about 0.01% w/w, about 0.05% w/w, about 0.1% w/w, about 0.2% w/w, about 0.3% w/w, about 0.4% w/w, about 0.46% w/w, about 0.5% w/w, about 0.6% w/w, about 0.7% w/w, about 0.8% w/w, about 0.9% w/w, about 1% w/w, about 2% w/w, about 3% w/w, or about 4% w/w. In some embodiments, the chewable gel product contains loratadine, or a pharmaceutically acceptable salt, ester, hydrate, or solvate thereof, in an amount that is about 0.46% w/w. In some embodiments, the chewable gel product contains loratadine, or a pharmaceutically acceptable salt, ester, hydrate, or solvate thereof, in an amount that is about 0.5% w/w. In some embodiments, the chewable gel product contains loratadine, or a pharmaceutically acceptable salt, ester, hydrate, or solvate thereof, in an amount that is about 2% w/w.

In some embodiments, the chewable gel product contains loratadine, or a pharmaceutically acceptable salt, ester, hydrate, or solvate thereof, in an amount that ranges from about 1 mg/ unit dose to about 50mg/ unit dose. In some embodiments, the chewable gel product contains loratadine, or a pharmaceutically acceptable salt, ester, hydrate, or solvate thereof, in an amount that is less than about 50 mg/ unit dose, less than about 40 mg/ unit dose, less than about 30 mg/ unit dose, less than about 25 mg/ unit dose, less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, or less than about 2 mg/ unit dose. In some embodiments, the chewable gel product contains loratadine, or a pharmaceutically acceptable salt, ester, hydrate, or solvate thereof, in an amount that is greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, greater than about 20 mg/ unit dose, greater than about 25 mg/ unit dose, greater than about 30 mg/ unit dose, or greater than about 40 mg/ unit dose. In some embodiments, the chewable gel product contains loratadine, or a pharmaceutically acceptable salt, ester, hydrate, or solvate thereof, in an amount that has a range of mg/ unit dose having an upper limit of about 50 mg/ unit dose, 40 mg/ unit dose, 30 mg/ unit dose, 25 mg/ unit dose, 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, or 2 mg/ unit dose, and an independently selected lower limit of about 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 15 mg/ unit dose, 20 mg/ unit dose, 25 mg/ unit dose, 30 mg/ unit dose, or 40 mg/ unit dose, where the lower limit is less than the upper limit. In some embodiments, the chewable gel product contains loratadine, or a pharmaceutically acceptable salt, ester, hydrate, or solvate thereof, in an amount of about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, about 25 mg/ unit dose, about 30 mg/ unit dose, about 40 mg/ unit dose, or about 50 mg/ unit dose. In some embodiments, the chewable gel product contains loratadine, or a pharmaceutically acceptable salt, ester, hydrate, or solvate thereof, an amount of about 10 mg/ unit dose.

In some embodiments, the chewable gel product contains diphenhydramine, or a pharmaceutically acceptable salt, ester, hydrate, or solvate thereof (e.g., diphenhydramine HCl, diphenhydramine citrate, etc.), in an amount that ranges from about0.01% w/w to about 50% w/w. In some embodiments, the chewable gel product contains diphenhydramine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains diphenhydramine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains diphenhydramine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains diphenhydramine, or a pharmaceutically acceptable salt, ester, hydrate, or solvate thereof, in an amount of at about 0.05% w/w, about 0.1% w/w, about 0.2% w/w, about 0.3% w/w, about 0.4% w/w, about 0.5% w/w, about 0.56% w/w, about 0.6% w/w, about 0.7% w/w, about 0.8% w/w, about 0.9% w/w, or about 1% w/w. In some embodiments, the chewable gel product contains diphenhydramine, or a pharmaceutically acceptable salt, ester, hydrate, or solvate thereof, in an amount of about 0.56% w/w.

In some embodiments, the chewable gel product contains diphenhydramine, or a pharmaceutically acceptable salt, ester, hydrate, or solvate thereof (e.g., diphenhydramine HCl, diphenhydramine citrate, etc.), in an amount that ranges from about 2 mg/ unit dose to about 100 mg/ unit dose. In some embodiments, the chewable gel product contains diphenhydramine, or a pharmaceutically acceptable salt, ester, hydrate, or solvate thereof, in an amount that is less than about 100 mg/ unit dose, less than about 75 mg/ unit dose, less than about 50 mg/ unit dose, less than about 40 mg/ unit dose, less than about 30 mg/ unit dose, less than about 25 mg/ unit dose, less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, or less than about 3 mg/ unit dose.. In some embodiments, the chewable gel product contains diphenhydramine, or a pharmaceutically acceptable salt, ester, hydrate, or solvate thereof, in an amount that is greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, greater than about 20 mg/ unit dose, greater than about 25 mg/ unit dose, greater than about 30 mg/ unit dose, greater than about 40 mg/ unit dose, greater than about 50 mg/ unit dose, or greater than about 75 mg/ unit dose. In some embodiments, the chewable gel product contains diphenhydramine, or a pharmaceutically acceptable salt, ester, hydrate, or solvate thereof, in an amount that has a range of mg/ unit dose having an upper limit of about 100 mg/ unit dose, 75 mg/ unit dose, 50 mg/ unit dose, 40 mg/ unit dose, 30 mg/ unit dose, 25 mg/ unit dose, 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, or 3 mg/ unit dose, and an independently selected lower limit of about 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 15 mg/ unit dose, 20 mg/ unit dose, 25 mg/ unit dose, 30 mg/ unit dose, 40 mg/ unit dose, 50 mg/ unit dose, or 75 mg/ unit dose, where the lower limit is less than the upper limit. In some embodiments, the chewable gel product contains diphenhydramine, or a pharmaceutically acceptable salt, ester, hydrate, or solvate thereof, in an amount of about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, about 25 mg/ unit dose, about 30 mg/ unit dose, about 40 mg/ unit dose, about 50 mg/ unit dose, about 75 mg/ unit dose, or about 100 mg/ unit dose. In some embodiments, the chewable gel product contains diphenhydramine, or a pharmaceutically acceptable salt, ester, hydrate, or solvate thereof, in an amount of about 25 mg/ unit dose.

In some embodiments, the chewable gel product contains desloratadine, or a pharmaceutically acceptable salt, ester, hydrate, or solvate thereof, in an amount that ranges from about 0.01% w/w to about 50% w/w. In some embodiments, the chewable gel product contains desloratadine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains desloratadine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains desloratadine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0. 1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains desloratadine, or a pharmaceutically acceptable salt, ester, hydrate, or solvate thereof, in an amount of at about 0.01% w/w, about 0.05% w/w, about 0.1% w/w, about 0.11% w/w, about 0.15% w/w, about 0.2% w/w, about 0.25% w/w, about 0.3% w/w, about 0.35% w/w, about 0.4% w/w, or about 0.5% w/w. In some embodiments, the chewable gel product contains desloratadine, or a pharmaceutically acceptable salt, ester, hydrate, or solvate thereof, in an amount of about 0.11% w/w.

In some embodiments, the chewable gel product contains desloratadine, or a pharmaceutically acceptable salt, ester, hydrate, or solvate thereof, in an amount that ranges from about 0.5 mg/ unit dose to about 25 mg/ unit dose. In some embodiments, the chewable gel product contains desloratadine, or a pharmaceutically acceptable salt, ester, hydrate, or solvate thereof, in an amount that is less than about 25 mg/ unit dose, less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, less than about 2 mg/ unit dose, less than about 1 mg/ unit dose, or less than about 0.75 mg/ unit dose. In some embodiments, the chewable gel product contains desloratadine, or a pharmaceutically acceptable salt, ester, hydrate, or solvate thereof, in an amount that is greater than about 0.5 mg/ unit dose, greater than about 0.75 mg/ unit dose, greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, or greater than about 20 mg/ unit dose. In some embodiments, the chewable gel product contains desloratadine, or a pharmaceutically acceptable salt, ester, hydrate, or solvate thereof, in an amount that has a range of mg/ unit dose having an upper limit of about 25 mg/ unit dose, 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, 2 mg/ unit dose, 1 mg/ unit dose, or 0.75 mg/ unit dose, and an independently selected lower limit of about 0.5 mg/ unit dose, 0.75 mg/ unit dose, 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 15 mg/ unit dose, or 20 mg/ unit dose, where the lower limit is less than the upper limit. In some embodiments, the chewable gel product contains desloratadine, or a pharmaceutically acceptable salt, ester, hydrate, or solvate thereof, in an amount of about 0.5 mg/ unit dose, about 0.75 mg/ unit dose, about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, or about 25 mg/ unit dose. In some embodiments, the chewable gel product contains desloratadine, or a pharmaceutically acceptable salt, ester, hydrate, or solvate thereof, in an amount of about 5 mg/ unit dose.

In some embodiments, the chewable gel product contains phenylephrine, or a pharmaceutically acceptable salt, ester, hydrate, or solvate thereof (e.g., phenylephrine HCl, phenylephrine bitartrate, etc.), in an amount that ranges from about 0.01% w/w to about 50% w/w. In some embodiments, the chewable gel product contains phenylephrine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains phenylephrine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains phenylephrine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains phenylephrine, or a pharmaceutically acceptable salt, ester, hydrate, or solvate thereof, in an amount of at about 0.01% w/w, about 0.05% w/w, about 0.1% w/w, about 0.11% w/w, about 0.15% w/w, about 0.2% w/w, about 0.25% w/w, about 0.3% w/w, about 0.35% w/w, about 0.4% w/w, or about 0.5% w/w. In some embodiments, the chewable gel product contains phenylephrine, or a pharmaceutically acceptable salt, ester, hydrate, or solvate thereof, in an amount of about 0.11% w/w.

In some embodiments, the chewable gel product contains phenylephrine, or a pharmaceutically acceptable salt, ester, hydrate, or solvate thereof (e.g., phenylephrine HCl, phenylephrine bitartrate, etc.), in an amount that ranges from about 1 mg/ unit dose to about 60 mg/ unit dose. In some embodiments, the chewable gel product contains phenylephrine, or a pharmaceutically acceptable salt, ester, hydrate, or solvate thereof, in an amount that is less than about 60 mg/ unit dose, less than about 50 mg/ unit dose, less than about 40 mg/ unit dose, less than about 30 mg/ unit dose, less than about 20 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, or less than about 2 mg/ unit dose. In some embodiments, the chewable gel product contains phenylephrine, or a pharmaceutically acceptable salt, ester, hydrate, or solvate thereof, in an amount that is greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 20 mg/ unit dose, greater than about 30 mg/ unit dose, greater than about 40 mg/ unit dose, or greater than about 50 mg/ unit dose. In some embodiments, the chewable gel product contains phenylephrine, or a pharmaceutically acceptable salt, ester, hydrate, or solvate thereof, in an amount that has a range of mg/ unit dose having an upper limit of about 60 mg/ unit dose, 50 mg/ unit dose, 40 mg/ unit dose, 30 mg/ unit dose, 20 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, or 2 mg/ unit dose, and an independently selected lower limit of about 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 20 mg/ unit dose, 30 mg/ unit dose, 40 mg/ unit dose, or 50 mg/ unit dose, where the lower limit is less than the upper limit. In some embodiments, the chewable gel product contains phenylephrine, or a pharmaceutically acceptable salt, ester, hydrate, or solvate thereof, in an amount of about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 20 mg/ unit dose, about 30 mg/ unit dose, about 40 mg/ unit dose, about 50 mg/ unit dose, or about 60 mg/ unit dose. In some embodiments, the chewable gel product contains phenylephrine, or a pharmaceutically acceptable salt, ester, hydrate, or solvate thereof, in an amount of about 5 mg/ unit dose.

In some embodiments, the chewable gel product contains chlorpheniramine, or a pharmaceutically acceptable salt, ester, hydrate, or solvate thereof (e.g., chlorpheniramine maleate, etc.), in an amount that ranges from about 0.01% w/w to about 50% w/w. In some embodiments, the chewable gel product contains chlorpheniramine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains chlorpheniramine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains chlorpheniramine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains chlorpheniramine, or a pharmaceutically acceptable salt, ester, hydrate, or solvate thereof, in an amount of about 0.01% w/w, about 0.05% w/w, about 0.1% w/w, about 0.11% w/w, about 0.15% w/w, about 0.2% w/w, about 0.25% w/w, about 0.3% w/w, about 0.35% w/w, about 0.4% w/w, or about 0.5% w/w. In some embodiments, the chewable gel product contains chlorpheniramine, or a pharmaceutically acceptable salt, ester, hydrate, or solvate thereof, in an amount of about 0.05% w/w.

In some embodiments, the chewable gel product contains chlorpheniramine, or a pharmaceutically acceptable salt, ester, hydrate, or solvate thereof (e.g., chlorpheniramine maleate, etc.), in an amount that ranges from about 0.5 mg/ unit dose to about 20 mg/ unit dose. In some embodiments, the chewable gel product contains chlorpheniramine, or a pharmaceutically acceptable salt, ester, hydrate, or solvate thereof, in an amount that is less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, less than about 2 mg/ unit dose, less than about 1 mg/ unit dose, or less than about 0.75 mg/ unit dose. In some embodiments, the chewable gel product contains chlorpheniramine, or a pharmaceutically acceptable salt, ester, hydrate, or solvate thereof, in an amount that is greater than about 0.5 mg/ unit dose, greater than about 0.75 mg/ unit dose, greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, or greater than about 15 mg/ unit dose. In some embodiments, the chewable gel product contains chlorpheniramine, or a pharmaceutically acceptable salt, ester, hydrate, or solvate thereof, in an amount that has a range of mg/ unit dose having an upper limit of about 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, 2 mg/ unit dose, 1 mg/ unit dose, or 0.75 mg/ unit dose, and an independently selected lower limit of about 0.5 mg/ unit dose, 0.75 mg/ unit dose, 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, or 15 mg/ unit dose, where the lower limit is less than the upper limit. In some embodiments, the chewable gel product contains chlorpheniramine, or a pharmaceutically acceptable salt, ester, hydrate, or solvate thereof, in an amount of about 0.5 mg/ unit dose, about 0.75 mg/ unit dose, about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, or about 20 mg/ unit dose. In some embodiments, the chewable gel product contains chlorpheniramine, or a pharmaceutically acceptable salt, ester, hydrate, or solvate thereof, in an amount of about 2.0 mg/ unit dose.

In some embodiments, the chewable gel product contains dextromethorphan, or a pharmaceutically acceptable salt, ester, hydrate, or solvate thereof (e.g., dextromethorphan HBr, etc.), in an amount that ranges from about 0.01% w/w to about 50% w/w. In some embodiments, the chewable gel product contains dextromethorphan, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains dextromethorphan, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains dextromethorphan, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w,1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains dextromethorphan, or a pharmaceutically acceptable salt, ester, hydrate, or solvate thereof, in an amount of 0.05% w/w, about 0.1% w/w, about 0.15% w/w, about 0.175% w/w, about 0.18% w/w, about 0.19% w/w, about 0.2% w/w, about 0.21% w/w, about 0.22% w/w, about 0.23% w/w, about 0.24% w/w, about 0.25% w/w, about 0.275% w/w, about 0.3% w/w, about 0.4% w/w, about 0.5% w/w, about 0.6% w/w, about 0.7% w/w, about 0.8% w/w, about 0.9% w/w, or about 1% w/w. In some embodiments, the chewable gel product contains dextromethorphan, or a pharmaceutically acceptable salt, ester, hydrate, or solvate thereof, in an amount of about 0.22% w/w.

In some embodiments, the chewable gel product contains dextromethorphan, or a pharmaceutically acceptable salt, ester, hydrate, or solvate thereof (e.g., dextromethorphan HBr, etc.), in an amount that ranges from about 1 mg/ unit dose to about 100 mg/ unit dose. In some embodiments, the chewable gel product contains dextromethorphan, or a pharmaceutically acceptable salt, ester, hydrate, or solvate thereof, in an amount that is less than about 100 mg/ unit dose, less than about 90 mg/ unit dose, less than about 80 mg/ unit dose, less than about 75 mg/ unit dose, less than about 70 mg/ unit dose, less than about 60 mg/ unit dose, less than about 50 mg/ unit dose, less than about 40 mg/ unit dose, less than about 30 mg/ unit dose, less than about 25 mg/ unit dose, less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, or less than about 2 mg/ unit dose. In some embodiments, the chewable gel product contains dextromethorphan, or a pharmaceutically acceptable salt, ester, hydrate, or solvate thereof, in an amount that is greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, greater than about 20 mg/ unit dose, greater than about 25 mg/ unit dose, greater than about 30 mg/ unit dose, greater than about 40 mg/ unit dose, greater than about 50 mg/ unit dose, greater than about 60 mg/ unit dose, greater than about 70 mg/ unit dose, greater than about 75 mg/ unit dose, greater than about 80 mg/ unit dose, or greater than about 90 mg/ unit dose. In some embodiments, the chewable gel product contains dextromethorphan, or a pharmaceutically acceptable salt, ester, hydrate, or solvate thereof, in an amount that is has a range of mg/ unit dose having an upper limit of about 100 mg/ unit dose, 90 mg/ unit dose, 80 mg/ unit dose, 75 mg/ unit dose,70 mg/ unit dose, 60 mg/ unit dose, 50 mg/ unit dose, 40 mg/ unit dose, 30 mg/ unit dose, 25 mg/ unit dose, 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, or 2 mg/ unit dose, and an independently selected lower limit of about 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 15 mg/ unit dose, 20 mg/ unit dose, 25 mg/ unit dose, 30 mg/ unit dose, 40 mg/ unit dose, 50 mg/ unit dose, 60 mg/ unit dose, 70 mg/ unit dose, 75 mg/ unit dose, 80 mg/ unit dose, or 90 mg/ unit dose, where the lower limit is less than the upper limit. In some embodiments, the chewable gel product contains dextromethorphan, or a pharmaceutically acceptable salt, ester, hydrate, or solvate thereof, in an amount that is about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, about 25 mg/ unit dose, about 30 mg/ unit dose, about 40 mg/ unit dose, about 50 mg/ unit dose, about 60 mg/ unit dose, about 70 mg/ unit dose, about 75 mg/ unit dose, about 80 mg/ unit dose, about 90 mg/ unit dose, or about 100 mg/ unit dose. In some embodiments, the chewable gel product contains dextromethorphan, or a pharmaceutically acceptable salt, ester, hydrate, or solvate thereof, in an amount that is about 10 mg/ unit dose.

In some embodiments, the chewable gel product contains doxylamine, or a pharmaceutically acceptable salt, ester, hydrate, or solvate thereof (e.g., doxylamine succinate, etc.), in an amount that ranges from about 0.01% w/w to about 50% w/w. In some embodiments, the chewable gel product contains doxylamine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains doxylamine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains doxylamine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w,1% w/w, 2% w/w, 3% w/w, 4% w/w, 5% w/w, 6% w/w, 7% w/w, 8% w/w, 9% w/w, 10% w/w, 15%'w/w, 20% w/w, 25% w/w, 30% w/w, 35% w/w, 40% w/w, or 45% w/w, where the lower limit is less than the upper limit. In some embodiments, the chewable gel product contains doxylamine, or a pharmaceutically acceptable salt, ester, hydrate, or solvate thereof, in an amount of at about 0.01% w/w, about 0.05% w/w, about 0.1% w/w, about 0.11% w/w, about 0.12% w/w, about 0.13% w/w, about 0.14% w/w, about 0.15% w/w, about 0.16% w/w, about 0.17% w/w, about 0.18% w/w, about 0.19% w/w, about 0.2% w/w, about 0.25% w/w, about 0.3% w/w, about 0.35% w/w, about 0.4% w/w, or about 0.5% w/w. In some embodiments, the chewable gel product contains doxylamine, or a pharmaceutically acceptable salt, ester, hydrate, or solvate thereof, in an amount of about 0.14% w/w.

In some embodiments, the chewable gel product contains doxylamine, or a pharmaceutically acceptable salt, ester, hydrate, or solvate thereof (e.g., doxylamine succinate, etc.), in an amount that ranges from about 5 mg/ unit dose to about 50 mg/ unit dose. In some embodiments, the chewable gel product contains doxylamine, or a pharmaceutically acceptable salt, ester, hydrate, or solvate thereof, in an amount that is less than about 50 mg/ unit dose, less than about 40 mg/ unit dose, less than about 30 mg/ unit dose, less than about 25 mg/ unit dose, less than about 20 mg/ unit dose, less than about 15 mg/ unit dose, or less than about 10 mg/ unit dose. In some embodiments, the chewable gel product contains doxylamine, or a pharmaceutically acceptable salt, ester, hydrate, or solvate thereof, in an amount that is greater than about 5 mg/ unit dose, greater than about 10 mg/ unit dose, greater than about 15 mg/ unit dose, greater than about 20 mg/ unit dose, greater than about 25 mg/ unit dose, greater than about 30 mg/ unit dose, or greater than about 40 mg/ unit dose. In some embodiments, the chewable gel product contains doxylamine, or a pharmaceutically acceptable salt, ester, hydrate, or solvate thereof, in an amount that has a range of mg/ unit dose having an upper limit of about 50 mg/ unit dose, 40 mg/ unit dose, 30 mg/ unit dose, 25 mg/ unit dose, 20 mg/ unit dose, 15 mg/ unit dose, or 10 mg/ unit dose, and an independently selected lower limit of about 5 mg/ unit dose, 10 mg/ unit dose, 15 mg/ unit dose, 20 mg/ unit dose, 25 mg/ unit dose, 30 mg/ unit dose, or 40 mg/ unit dose, where the lower limit is less than the upper limit. In some embodiments, the chewable gel product contains doxylamine, or a pharmaceutically acceptable salt, ester, hydrate, or solvate thereof, in an amount of about 5 mg/ unit dose, about 10 mg/ unit dose, about 15 mg/ unit dose, about 20 mg/ unit dose, about 25 mg/ unit dose, about 30 mg/ unit dose, about 40 mg/ unit dose, or about 50 mg/ unit dose. In some embodiments, the chewable gel product contains doxylamine, or a pharmaceutically acceptable salt, ester, hydrate, or solvate thereof, in an amount of about 6.25 mg/ unit dose.

In some embodiments, the chewable gel product contains guaifenesin, or a pharmaceutically acceptable salt, ester, hydrate, or solvate thereof, in an amount that ranges from about 0.01% w/w to about 50% w/w. In some embodiments, the chewable gel product contains guaifenesin, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, or less than about 0.1% w/w, less than about 0.05% w/w, or less than about 0.01% w/w. In some embodiments, the chewable gel product contains guaifenesin, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains guaifenesin, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains guaifenesin, or a pharmaceutically acceptable salt, ester, hydrate, or solvate thereof, in an amount of about 1% w/w, about 2% w/w, about 3% w/w, about 3.5% w/w, about 3.75% w/w, about 4% w/w, about 4.1% w/w, about 4.2% w/w, about 4.3% w/w, about 4.4% w/w, about 4.5% w/w, about 4.6% w/w, about 4.7% w/w, about 5% w/w, about 6% w/w, about 7% w/w, about 8% w/w, about 9% w/w, or about 10% w/w. In some embodiments, the chewable gel product contains guaifenesin, or a pharmaceutically acceptable salt, ester, hydrate, or solvate thereof, in an amount of about 4.3% w/w.

In some embodiments, the chewable gel product contains guaifenesin, or a pharmaceutically acceptable salt, ester, hydrate, or solvate thereof, in an amount that ranges from about 10 mg/ unit dose to about 1,000 mg/ unit dose. In some embodiments, the chewable gel product contains guaifenesin, or a pharmaceutically acceptable salt, ester, hydrate, or solvate thereof, in an amount that is less than about 1,000 mg/ unit dose, less than about 750 mg/ unit dose, less than about 500 mg/ unit dose, less than about 400 mg/ unit dose, less than about 300 mg/ unit dose, less than about 200 mg/ unit dose, less than about 100 mg/ unit dose, less than about 75 mg/ unit dose, less than about 50 mg/ unit dose, less than about 25 mg/ unit dose, or less than about 15 mg/ unit dose. In some embodiments, the chewable gel product contains guaifenesin, or a pharmaceutically acceptable salt, ester, hydrate, or solvate thereof, in an amount that is greater than about 10 mg/ unit dose, greater than about 25 mg/ unit dose, greater than about 50 mg/ unit dose, greater than about 75 mg/ unit dose, greater than about 100 mg/ unit dose, greater than about 200 mg/ unit dose, greater than about 300 mg/ unit dose, greater than about 400 mg/ unit dose, greater than about 500 mg/ unit dose, greater than about 750 mg/ unit dose, or greater than about 900 mg/ unit dose. In some embodiments, the chewable gel product contains guaifenesin, or a pharmaceutically acceptable salt, ester, hydrate, or solvate thereof, in an amount that has a range of mg/ unit dose having an upper limit of about 1,000 mg/ unit dose, 750 mg/ unit dose, 500 mg/ unit dose, 400 mg/ unit dose, 300 mg/ unit dose, 200 mg/ unit dose, 100 mg/ unit dose, 75 mg/ unit dose, 50 mg/ unit dose, 25 mg/ unit dose, or 15 mg/ unit dose, and an independently selected lower limit of about 10 mg/ unit dose, 25 mg/ unit dose, 50 mg/ unit dose, 75 mg/ unit dose, 100 mg/ unit dose, 200 mg/ unit dose, 300 mg/ unit dose, 400 mg/ unit dose, 500 mg/ unit dose, 750 mg/ unit dose, or 900 mg/ unit dose, where the lower limit is less than the upper limit. In some embodiments, the chewable gel product contains guaifenesin, or a pharmaceutically acceptable salt, ester, hydrate, or solvate thereof, in an amount of about 10 mg/ unit dose, about 25 mg/ unit dose, about 50 mg/ unit dose, about 75 mg/ unit dose, about 100 mg/ unit dose, about 200 mg/ unit dose, about 300 mg/ unit dose, about 400 mg/ unit dose, about 500 mg/ unit dose, about 750 mg/ unit dose, or about 1,000 mg/ unit dose. In some embodiments, the chewable gel product contains guaifenesin, or a pharmaceutically acceptable salt, ester, hydrate, or solvate thereof, in an amount of about 200 mg/ unit dose.

In some embodiments, the chewable gel product contains fexofenadine, or a pharmaceutically acceptable salt, ester, hydrate, or solvate thereof (e.g., fexofenadine HCl, etc.), in an amount that ranges from about 0.01% w/w to about 50% w/w. In some embodiments, the chewable gel product contains fexofenadine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains fexofenadine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains fexofenadine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains fexofenadine, or a pharmaceutically acceptable salt, ester, hydrate, or solvate thereof, in an amount of at about 0.1% w/w, about 0.5% w/w, about 0.75% w/w, about 1% w/w, about 1.1% w/w, about 1.2% w/w, about 1.3% w/w, about 1.33% w/w, about 1.36% w/w, about 1.4% w/w, about 1.5% w/w, about 1.75% w/w, about 2% w/w, about 2.5% w/w, about 3% w/w, about 3.5% w/w, about 4% w/w, about 4.5% w/w, or about 5% w/w. In some embodiments, the chewable gel product contains fexofenadine, or a pharmaceutically acceptable salt, ester, hydrate, or solvate thereof, in an amount of about 1.33% w/w.

In some embodiments, the chewable gel product contains fexofenadine, or a pharmaceutically acceptable salt, ester, hydrate, or solvate thereof (e.g., fexofenadine HCl, etc.), in an amount that ranges from about 15 mg/ unit dose to about 360 mg/ unit dose. In some embodiments, the chewable gel product contains fexofenadine, or a pharmaceutically acceptable salt, ester, hydrate, or solvate thereof, in an amount that is less than about 360 mg/ unit dose, less than about 300 mg/ unit dose, less than about 250 mg/ unit dose, less than about 200 mg/ unit dose, less than about 150 mg/ unit dose, less than about 100 mg/ unit dose, less than about 75 mg/ unit dose, less than about 50 mg/ unit dose, less than about 40 mg/ unit dose, less than about 30 mg/ unit dose, less than about 25 mg/ unit dose, or less than about 20 mg/ unit dose. In some embodiments, the chewable gel product contains fexofenadine, or a pharmaceutically acceptable salt, ester, hydrate, or solvate thereof, in an amount that is greater than about 15 mg/ unit dose, greater than about 20 mg/ unit dose, greater than about 25 mg/ unit dose, greater than about 30 mg/ unit dose, greater than about 40 mg/ unit dose, greater than about 50 mg/ unit dose, greater than about 75 mg/ unit dose, greater than about 100 mg/ unit dose, greater than about 150 mg/ unit dose, greater than about 200 mg/ unit dose, greater than about 250 mg/ unit dose, greater than about 300 mg/ unit dose, or greater than about 350 mg/ unit dose. In some embodiments, the chewable gel product contains fexofenadine, or a pharmaceutically acceptable salt, ester, hydrate, or solvate thereof, in an amount that has a range of mg/ unit dose having an upper limit of about 360 mg/ unit dose, 300 mg/ unit dose, 250 mg/ unit dose, 200 mg/ unit dose, 150 mg/ unit dose, 100 mg/ unit dose, 75 mg/ unit dose, 50 mg/ unit dose, 40 mg/ unit dose, 30 mg/ unit dose, 25 mg/ unit dose, or 20 mg/ unit dose, and an independently selected lower limit of about 15 mg/ unit dose, 20 mg/ unit dose, 25 mg/ unit dose, 30 mg/ unit dose, 40 mg/ unit dose, 50 mg/ unit dose, 75 mg/ unit dose, 100 mg/ unit dose, 150 mg/ unit dose, 200 mg/ unit dose, 250 mg/ unit dose, 300 mg/ unit dose, or 350 mg/ unit dose, where the lower limit is less than the upper limit. In some embodiments, the chewable gel product contains fexofenadine, or a pharmaceutically acceptable salt, ester, hydrate, or solvate thereof, in an amount of about 15 mg/ unit dose, about 20 mg/ unit dose, about 25 mg/ unit dose, about 30 mg/ unit dose, about 40 mg/ unit dose, about 50 mg/ unit dose, about 75 mg/ unit dose, about 100 mg/ unit dose, about 150 mg/ unit dose, about 200 mg/ unit dose, about 250 mg/ unit dose, about 300 mg/ unit dose, or about 360 mg/ unit dose. In some embodiments, the chewable gel product contains fexofenadine, or a pharmaceutically acceptable salt, ester, hydrate, or solvate thereof, in an amount of about 60 mg/ unit dose.

In some embodiments, the chewable gel product contains docusate, or a pharmaceutically acceptable salt, ester, hydrate, or solvate thereof (e.g., docusate sodium, etc.), in an amount that ranges from about 0.01% w/w to about 50% w/w. In some embodiments, the chewable gel product contains docusate, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains docusate, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains docusate, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains docusate, or a pharmaceutically acceptable salt, ester, hydrate, or solvate thereof, in an amount of at about 0.1% w/w, about 0.5% w/w, about 0.75% w/w, about 0.8% w/w, about 0.9% w/w, about 1% w/w, about 1.1% w/w, about 1.2% w/w, about 1.3% w/w, about 1.4% w/w, about 1.5% w/w, about 1.75% w/w, about 2% w/w, about 2.5% w/w, about 3% w/w, about 3.5% w/w, about 4% w/w, about 4.5% w/w, or about 5% w/w . In some embodiments, the chewable gel product contains docusate, or a pharmaceutically acceptable salt, ester, hydrate, or solvate thereof, in an amount of about 1.1% w/w.

In some embodiments, the chewable gel product contains docusate, or a pharmaceutically acceptable salt, ester, hydrate, or solvate thereof (e.g., docusate sodium, etc.), in an amount that ranges from about 5 mg/ unit dose to about 360 mg/ unit dose. In some embodiments, the chewable gel product contains docusate, or a pharmaceutically acceptable salt, ester, hydrate, or solvate thereof, in an amount that is less than about 360 mg/ unit dose, less than about 300 mg/ unit dose, less than about 250 mg/ unit dose, less than about 200 mg/ unit dose, less than about 150 mg/ unit dose, less than about 100 mg/ unit dose, less than about 75 mg/ unit dose, less than about 50 mg/ unit dose, less than about 40 mg/ unit dose, less than about 30 mg/ unit dose, less than about 25 mg/ unit dose, less than about 20 mg/ unit dose, less than about 15 mg/ unit dose, or less than about 10 mg/ unit dose. In some embodiments, the chewable gel product contains docusate, or a pharmaceutically acceptable salt, ester, hydrate, or solvate thereof, in an amount that is greater than about 5 mg/ unit dose, greater than about 10 mg/ unit dose, greater than about 15 mg/ unit dose, greater than about 20 mg/ unit dose, greater than about 25 mg/ unit dose, greater than about 30 mg/ unit dose, greater than about 40 mg/ unit dose, greater than about 50 mg/ unit dose, greater than about 75 mg/ unit dose, greater than about 100 mg/ unit dose, greater than about 150 mg/ unit dose, greater than about 200 mg/ unit dose, greater than about 250 mg/ unit dose, greater than about 300 mg/ unit dose, or greater than about 350 mg/ unit dose. In some embodiments, the chewable gel product contains docusate, or a pharmaceutically acceptable salt, ester, hydrate, or solvate thereof, in an amount that has a range of mg/ unit dose having an upper limit of about 360 mg/ unit dose, 300 mg/ unit dose, 250 mg/ unit dose, 200 mg/ unit dose, 150 mg/ unit dose, 100 mg/ unit dose, 75 mg/ unit dose, 50 mg/ unit dose, 40 mg/ unit dose, 30 mg/ unit dose, 25 mg/ unit dose, 20 mg/ unit dose, 15 mg/ unit dose, or 10 mg/ unit dose, and an independently selected lower limit of about 5 mg/ unit dose, 10 mg/ unit dose, 15 mg/ unit dose, 20 mg/ unit dose, 25 mg/ unit dose, 30 mg/ unit dose, 40 mg/ unit dose, 50 mg/ unit dose, 75 mg/ unit dose, 100 mg/ unit dose, 150 mg/ unit dose, 200 mg/ unit dose, 250 mg/ unit dose, 300 mg/ unit dose, or 350 mg/ unit dose, where the lower limit is less than the upper limit. In some embodiments, the chewable gel product contains docusate, or a pharmaceutically acceptable salt, ester, hydrate, or solvate thereof, in an amount of about 5 mg/ unit dose, about 10 mg/ unit dose, about 15 mg/ unit dose, about 20 mg/ unit dose, about 25 mg/ unit dose, about 30 mg/ unit dose, about 40 mg/ unit dose, about 50 mg/ unit dose, about 75 mg/ unit dose, about 100 mg/ unit dose, about 150 mg/ unit dose, about 200 mg/ unit dose, about 250 mg/ unit dose, about 300 mg/ unit dose, or about 360 mg/ unit dose. In some embodiments, the chewable gel product contains docusate, or a pharmaceutically acceptable salt, ester, hydrate, or solvate thereof, in an amount of about 50 mg/ unit dose.

In some embodiments, the chewable gel product contains pseudoephedrine, or a pharmaceutically acceptable salt, ester, hydrate, or solvate thereof (e.g., pseudoephedrine HCl, pseudoephedrine sulfate, etc.), in an amount that ranges from about 0.01% w/w to about 50% w/w. In some embodiments, the chewable gel product contains pseudoephedrine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains pseudoephedrine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains pseudoephedrine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains pseudoephedrine, or a pharmaceutically acceptable salt, ester, hydrate, or solvate thereof, in an amount of at about 0.01% w/w, about 0.25% w/w, about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 15% w/w, about 20% w/w, about 25% w/w, about 30% w/w, about 35% w/w, about 40% w/w, about 45% w/w, or about 50% w/w.

In some embodiments, the chewable gel product contains pseudoephedrine, or a pharmaceutically acceptable salt, ester, hydrate, or solvate thereof (e.g., pseudoephedrine HCl, pseudoephedrine sulfate, etc.), in an amount that ranges from about 6 mg/ unit dose to about 240 mg/ unit dose. In some embodiments, the chewable gel product contains pseudoephedrine, or a pharmaceutically acceptable salt, ester, hydrate, or solvate thereof, in an amount that is less than about 240 mg/ unit dose, less than about 200 mg/ unit dose, less than about 150 mg/ unit dose, less than about 100 mg/ unit dose, less than about 75 mg/ unit dose, less than about 50 mg/ unit dose, less than about 25 mg/ unit dose, less than about 20 mg/ unit dose, less than about 15 mg/ unit dose, less than about 10 mg/ unit dose, less than about 9 mg/ unit dose, less than about 8 mg/ unit dose, or less than about 7 mg/ unit dose. In some embodiments, the chewable gel product contains pseudoephedrine, or a pharmaceutically acceptable salt, ester, hydrate, or solvate thereof, in an amount that is greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, greater than about 20 mg/ unit dose, greater than about 25 mg/ unit dose, greater than about 50 mg/ unit dose, greater than about 75 mg/ unit dose, greater than about 100 mg/ unit dose, greater than about 150 mg/ unit dose, greater than about 200 mg/ unit dose, or greater than about 225 mg/ unit dose. In some embodiments, the chewable gel product contains pseudoephedrine, or a pharmaceutically acceptable salt, ester, hydrate, or solvate thereof, in an amount that has a range of mg/ unit dose having an upper limit of about 240 mg/ unit dose, 200 mg/ unit dose, 150 mg/ unit dose, 100 mg/ unit dose, 75 mg/ unit dose, 50 mg/ unit dose, 25 mg/ unit dose, 20 mg/ unit dose, 15 mg/ unit dose, 10 mg/ unit dose, 9 mg/ unit dose, 8 mg/ unit dose, or 7 mg/ unit dose, and an independently selected lower limit of about 6 mg/ unit dose, 7 mg/ unit dose, 8 mg/ unit dose, 9 mg/ unit dose, 10 mg/ unit dose, 15 mg/ unit dose, 20 mg/ unit dose, 25 mg/ unit dose, 50 mg/ unit dose, 75 mg/ unit dose, 100 mg/ unit dose, 150 mg/ unit dose, 200 mg/ unit dose, or 225 mg/ unit dose, where the lower limit is less than the upper limit. In some embodiments, the chewable gel product contains pseudoephedrine, or a pharmaceutically acceptable salt, ester, hydrate, or solvate thereof, in an amount of about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, about 25 mg/ unit dose, about 50 mg/ unit dose, about 75 mg/ unit dose, about 100 mg/ unit dose, about 150 mg/ unit dose, about 200 mg/ unit dose, or about 240 mg/ unit dose. In some embodiments, the chewable gel product contains pseudoephedrine, or a pharmaceutically acceptable salt, ester, hydrate, or solvate thereof, in an amount of about 60 mg/ unit dose.

In some embodiments, the chewable gel product contains cetirizine, or a pharmaceutically acceptable salt, ester, hydrate, or solvate thereof (e.g., cetirizine HCl, etc.), in an amount that ranges from about 0.01% w/w to about 50% w/w. In some embodiments, the chewable gel product contains cetirizine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains cetirizine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains cetirizine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains cetirizine, or a pharmaceutically acceptable salt, ester, hydrate, or solvate thereof, in an amount of at about 0.01% w/w, about 0.25% w/w, about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 15% w/w, about 20% w/w, about 25% w/w, about 30% w/w, about 35% w/w, about 40% w/w, about 45% w/w, or about 50% w/w.

In some embodiments, the chewable gel product contains cetirizine, or a pharmaceutically acceptable salt, ester, hydrate, or solvate thereof (e.g., cetirizine HCl, etc.), in an amount that ranges from about 2 mg/ unit dose to about 30 mg/ unit dose. In some embodiments, the chewable gel product contains cetirizine, or a pharmaceutically acceptable salt, ester, hydrate, or solvate thereof, in an amount that is less than about 30 mg/ unit dose, less than about 25 mg/ unit dose, less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, or less than about 3 mg/ unit dose. In some embodiments, the chewable gel product contains cetirizine, or a pharmaceutically acceptable salt, ester, hydrate, or solvate thereof, in an amount that is greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, greater than about 20 mg/ unit dose, or greater than about 25 mg/ unit dose. In some embodiments, the chewable gel product contains cetirizine, or a pharmaceutically acceptable salt, ester, hydrate, or solvate thereof, in an amount that has a range of mg/ unit dose having an upper limit of about 30 mg/ unit dose, 25 mg/ unit dose, 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, or 3 mg/ unit dose, and an independently selected lower limit of about 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 15 mg/ unit dose, 20 mg/ unit dose, or 25 mg/ unit dose, where the lower limit is less than the upper limit. In some embodiments, the chewable gel product contains cetirizine, or a pharmaceutically acceptable salt, ester, hydrate, or solvate thereof, in an amount of about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, about 25 mg/ unit dose, or about 30 mg/ unit dose. In some embodiments, the chewable gel product contains cetirizine, or a pharmaceutically acceptable salt, ester, hydrate, or solvate thereof, in an amount of about 10 mg/ unit dose.

In some embodiments, the chewable gel product contains triprolidine, or a pharmaceutically acceptable salt, ester, hydrate, or solvate thereof (e.g., triprolidine HCl, etc.), in an amount that ranges from about 0.01% w/w to about 50% w/w. In some embodiments, the chewable gel product contains triprolidine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, less than about 0.1% w/w, less than about 0.05% w/w, or less than about 0.01% w/w. In some embodiments, the chewable gel product contains triprolidine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains triprolidine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.05% w/w, 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains triprolidine, or a pharmaceutically acceptable salt, ester, hydrate, or solvate thereof, in an amount of at about 0.01% w/w, about 0.25% w/w, about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 15% w/w, about 20% w/w, about 25% w/w, about 30% w/w, about 35% w/w, about 40% w/w, about 45% w/w, or about 50% w/w.

In some embodiments, the chewable gel product contains triprolidine, or a pharmaceutically acceptable salt, ester, hydrate, or solvate thereof (e.g., triprolidine HCl, etc.), in an amount that ranges from about 0.2 mg/ unit dose to about 10 mg/ unit dose. In some embodiments, the chewable gel product contains triprolidine, or a pharmaceutically acceptable salt, ester, hydrate, or solvate thereof, in an amount that is less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, less than about 2 mg/ unit dose, less than about 1.5 mg/ unit dose, less than about 1 mg/ unit dose, less than about 0.9 mg/ unit dose, less than about 0.8 mg/ unit dose, less than about 0.7 mg/ unit dose, less than about 0.6 mg/ unit dose, less than about 0.5 mg/ unit dose, less than about 0.4 mg/ unit dose, or less than about 0.3 mg/ unit dose. In some embodiments, the chewable gel product contains triprolidine, or a pharmaceutically acceptable salt, ester, hydrate, or solvate thereof, in an amount that is greater than about greater than about 0.2 mg/ unit dose, greater than about 0.3 mg/ unit dose, greater than about 0.4 mg/ unit dose, greater than about 0.5 mg/ unit dose, greater than about 0.6 mg/ unit dose, greater than about 0.7 mg/ unit dose, greater than about 0.8 mg/ unit dose, greater than about 0.9 mg/ unit dose, greater than about 1 mg/ unit dose, greater than about 1.5 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, or greater than about 9 mg/ unit dose. In some embodiments, the chewable gel product contains triprolidine, or a pharmaceutically acceptable salt, ester, hydrate, or solvate thereof, in an amount that has a range of mg/ unit dose having an upper limit of about 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, 2 mg/ unit dose, 1.5 mg/ unit dose, 1 mg/ unit dose, 0.9 mg/ unit dose, 0.8 mg/ unit dose, 0.7 mg/ unit dose, 0.6 mg/ unit dose, 0.5 mg/ unit dose, 0.4 mg/ unit dose, or 0.3 mg/ unit dose, and an independently selected lower limit of about 0.2 mg/ unit dose, 0.3 mg/ unit dose, 0.4 mg/ unit dose, 0.5 mg/ unit dose, 0.6 mg/ unit dose, 0.7 mg/ unit dose, 0.8 mg/ unit dose, 0.9 mg/ unit dose, 1 mg/ unit dose, 1.5 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, or 9 mg/ unit dose, where the lower limit is less than the upper limit. In some embodiments, the chewable gel product contains triprolidine, or a pharmaceutically acceptable salt, ester, hydrate, or solvate thereof, in an amount of about 0.2 mg/ unit dose, about 0.3 mg/ unit dose, about 0.4 mg/ unit dose, about 0.5 mg/ unit dose, about 0.6 mg/ unit dose, about 0.7 mg/ unit dose, about 0.8 mg/ unit dose, about 0.9 mg/ unit dose, about 1 mg/ unit dose, about 1.5 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, or about 10 mg/ unit dose. In some embodiments, the chewable gel product contains triprolidine, or a pharmaceutically acceptable salt, ester, hydrate, or solvate thereof, in an amount of about 2.5 mg/ unit dose.

In some embodiments, the chewable gel product contains brompheniramine, or a pharmaceutically acceptable salt, ester, hydrate, or solvate thereof (e.g., brompheniramine maleate, etc.), in an amount that ranges from about 0.01% w/w to about 50% w/w. In some embodiments, the chewable gel product contains brompheniramine, or a pharmaceutically acceptable salt, ester, hydrate, or solvate thereof, in an amount that is less than about 50% w/w, less than about 40% w/w, less than about 35% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, or less than about 0.1% w/w. In some embodiments, the chewable gel product contains brompheniramine, or a pharmaceutically acceptable salt, ester, hydrate, or solvate thereof, in an amount that is greater than about 0.01% w/w, greater than about 0.05% w/w, greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 35% w/w, greater than about 40% w/w, or greater than about 45% w/w. In some embodiments, the chewable gel product contains brompheniramine, or a pharmaceutically acceptable salt, ester, hydrate, or solvate thereof, in an amount that has a range of % w/w having an upper limit of about 50% w/w, 45% w/w, 40% w/w, 35% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.01% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 35% w/w, 40% w/w, or 45% w/w, where the lower limit is less than the upper limit. In some embodiments, the chewable gel product contains brompheniramine, or a pharmaceutically acceptable salt, ester, hydrate, or solvate thereof, in an amount of at about 0.01% w/w, about 0.25% w/w, about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 15% w/w, about 20% w/w, about 25% w/w, about 30% w/w, about 35% w/w, about 40% w/w, about 45% w/w, or about 50% w/w.

In some embodiments, the chewable gel product contains brompheniramine, or a pharmaceutically acceptable salt, ester, hydrate, or solvate thereof (e.g., brompheniramine maleate, etc.), in an amount that ranges from about 0.4 mg/ unit dose to about 60 mg/ unit dose. In some embodiments, the chewable gel product contains brompheniramine, or a pharmaceutically acceptable salt, ester, hydrate, or solvate thereof, in an amount that is less than about less than about 60 mg/ unit dose, less than about 50 mg/ unit dose, less than about 40 mg/ unit dose, less than about 30 mg/ unit dose, less than about 20 mg/ unit dose, less than about 15 mg/ unit dose, less than about 10 mg/ unit dose, less than about 9 mg/ unit dose, less than about 8 mg/ unit dose, less than about 7 mg/ unit dose, less than about 6 mg/ unit dose, less than about 5 mg/ unit dose, less than about 4 mg/ unit dose, less than about 3 mg/ unit dose, less than about 2 mg/ unit dose, less than about 1 mg/ unit dose, less than about 0.8 mg/ unit dose, or less than about 0.6 mg/ unit dose. In some embodiments, the chewable gel product contains brompheniramine, or a pharmaceutically acceptable salt, ester, hydrate, or solvate thereof, in an amount that is greater than about 0.4 mg/ unit dose, greater than about 0.6 mg/ unit dose, greater than about 0.8 mg/ unit dose, greater than about 1 mg/ unit dose, greater than about 2 mg/ unit dose, greater than about 3 mg/ unit dose, greater than about 4 mg/ unit dose, greater than about 5 mg/ unit dose, greater than about 6 mg/ unit dose, greater than about 7 mg/ unit dose, greater than about 8 mg/ unit dose, greater than about 9 mg/ unit dose, greater than about 10 mg/ unit dose, greater than about 15 mg/ unit dose, greater than about 20 mg/ unit dose, greater than about 30 mg/ unit dose, greater than about 40 mg/ unit dose, or greater than about 50 mg/ unit dose. In some embodiments, the chewable gel product contains brompheniramine, or a pharmaceutically acceptable salt, ester, hydrate, or solvate thereof, in an amount that has a range of mg/ unit dose having an upper limit of about 60 mg/ unit dose, 50 mg/ unit dose, 40 mg/ unit dose, 30 mg/ unit dose, 20 mg/ unit dose, 15 mg/ unit dose, 10 mg/ unit dose, 9 mg/ unit dose, 8 mg/ unit dose, 7 mg/ unit dose, 6 mg/ unit dose, 5 mg/ unit dose, 4 mg/ unit dose, 3 mg/ unit dose, 2 mg/ unit dose, 1 mg/ unit dose, 0.8 mg/ unit dose, or 0.6 mg/ unit dose, and an independently selected lower limit of about 0.4 mg/ unit dose, 0.6 mg/ unit dose, 0.8 mg/ unit dose, 1 mg/ unit dose, 2 mg/ unit dose, 3 mg/ unit dose, 4 mg/ unit dose, 5 mg/ unit dose, 6 mg/ unit dose, 7 mg/ unit dose, 8 mg/ unit dose, 9 mg/ unit dose, 10 mg/ unit dose, 15 mg/ unit dose, 20 mg/ unit dose, 30 mg/ unit dose, 40 mg/ unit dose, or 50 mg/ unit dose, where the lower limit is less than the upper limit. In some embodiments, the chewable gel product contains brompheniramine, or a pharmaceutically acceptable salt, ester, hydrate, or solvate thereof, in an amount of about 0.4 mg/ unit dose, about 0.6 mg/ unit dose, about 0.8 mg/ unit dose, about 1 mg/ unit dose, about 2 mg/ unit dose, about 3 mg/ unit dose, about 4 mg/ unit dose, about 5 mg/ unit dose, about 6 mg/ unit dose, about 7 mg/ unit dose, about 8 mg/ unit dose, about 9 mg/ unit dose, about 10 mg/ unit dose, about 15 mg/ unit dose, about 20 mg/ unit dose, about 30 mg/ unit dose, about 40 mg/ unit dose, about 50 mg/ unit dose, or about 60 mg/ unit dose.

### Taste Acceptability Index

In some aspects, the present disclosure relates to chewable gel products formulated such that the final chewable gel products have a palatable and non-bitter taste profile. In some embodiments, the chewable gel products are formulated to have a palatable and non-bitter taste profile through the use of acid-activated gel carriers formulated at precise pH ranges using only taste compatible pH adjusting agents, or through the use of non-acid-activated gel carriers. In some embodiments, the bitterness taste profile of the chewable gel product may be measured by a taste acceptability index (TAI).

As disclosed herein, the taste acceptability index (TAI) of a chewable gel product of the present disclosure may be assigned by a panel of tasters. The TAI may be calculated by determining the presence and quantity of bitterness of an API-containing chewable gel product by each taster, assigning a numerical value to each chewable gel product based on the degree of bitterness detected in the formulation scaled from 0 (extremely bitter and unpalatable taste profile) to 10 (no bitterness detected), and averaging the scores from each taster on the panel to identify the TAI of the given chewable gel product. In some embodiments, a TAI score of about 0 to about 4 indicates a chewable gel product that has an extreme or high level of bitter and/or unpalatable taste profiles. In some embodiments, a TAI score of about 5 to about 6 indicates a chewable gel product that has a moderate to low level of bitter and/or unpalatable taste profiles. In some embodiments, a score of about 7 to about 10 indicates a chewable gel product that has little to no bitter and/or unpalatable taste profiles. In some embodiments, TAI may be measured prior to gelation of the chewable gel product, for example, while the product is still in a liquid state. In some embodiments, TAI may be measured prior to formulation with an acid-activated gel carrier or prior to formulation with a non-acid-activated gel carrier. In some embodiments, TAI may be measured after gelation of the chewable gel product.

In some embodiments, the chewable gel product may be formulated to yield an API-containing composition having a taste acceptability index (TAI) of about 6 to about 10. Chewable gel products of the present disclosure can be formulated such that the product is not unpalatable but concurrently does not have a taste profile similar to a confectionary product. Additionally, the chewable gel product can be formulated such that a slight bitter taste is detectable, which may preferable to some individuals. Accordingly, in some embodiments, the chewable gel product may be formulated to yield an API-containing composition having a taste acceptability index (TAI) of about 6 to about 8.

In some embodiments, the chewable gel product has a TAI of about 6 to about 10, about 6 to about 9, about 6 to about 8, about 6 to about 7, about 7 to about 10, about 7 to about 9, about 7 to about 8, about 8 to about 10, about 8 to about 9, or about 9 to about 10. In some embodiments, the chewable gel product has a TAI of about 6 to about 8, about 6 to about 7, or about 7 to about 8. In some embodiments, the chewable gel product has a TAI of greater than about 5, greater than about 5.1, greater than about 5.2, greater than about 5.3, greater than about 5.4, greater than about 5.5, greater than about 5.6, greater than about 5.7, greater than about 5.8, greater than about 5.9, greater than about 6, greater than about 6.1, greater than about 6.2, greater than about 6.3, greater than about 6.4, greater than about 6.5, greater than about 6.6, greater than about 6.7, greater than about 6.8, greater than about 6.9,greater than about 7, greater than about 7.1, greater than about 7.2, greater than about 7.3, greater than about 7.4, greater than about 7.5, greater than about 7.6, greater than about 7.7, greater than about 7.8, greater than about 7.9, greater than about 8, greater than about 8. 1, greater than about 8.2, greater than about 8.3, greater than about 8.4, greater than about 8.5, greater than about 8.6, greater than about 8.7, greater than about 8.8, greater than about 8.9, or greater than about 9. In some embodiments, the chewable gel product has a TAI of about 6, about 6.1, about 6.2, about 6.3, about 6.4, about 6.5, about 6.6, about 6.7, about 6.8, about 6.9,about 7, about 7.1, about 7.2, about 7.3, about 7.4, about 7.5, about 7.6, about 7.7, about 7.8, about 7.9, about 8, about 8.1, about 8.2, about 8.3, about 8.4, about 8.5, about 8.6, about 8.7, about 8.8, about 8.9, about 9, about 9. 1, about 9.2, about 9.3, about 9.4, about 9.5, about 9.6, about 9.7, about 9.8, about 9.9, or about 10. In some embodiments, the chewable gel product has a TAI of at least about 6. In some embodiments, the chewable gel product has a TAI of at least about 7. In some embodiments, the chewable gel product has a TAI of at least about 8. In some embodiments, the chewable gel product has a TAI of at least about 9. In some embodiments, the chewable gel product has a TAI of about 10.

### Wetting Agents

In some aspects, the present disclosure relates to chewable gel products containing one or more *(e.g.,* one or more, two or more, three or more, four or more, five or more, *etc.)* wetting agents. In some embodiments, the one or more wetting agents are capable of dispersing/evenly dispersing an API in a chewable gel product of the present disclosure. The one or more wetting agents may be any suitable wetting agent, including, for example, propylene glycol, propylene glycol alginate, glycerin, sorbitol, polyethylene glycol, maltitol syrup, lecithin, and any combination thereof. It is to be understood that any of the wetting agents of the present disclosure may be provided in a solid form or in a solution form that included the solid dissolved into a solution. In some embodiments, the wetting agent is propylene glycol. In some embodiments, the wetting agent is propylene glycol alginate. In some embodiments, the wetting agent is glycerin. In some embodiments, the wetting agent is sorbitol. In some embodiments, the wetting agent is polyethylene glycol. In some embodiments, the wetting agent is sorbitol. In some embodiments, the wetting agent is maltitol syrup. In some embodiments, the wetting agent is sorbitol. In some embodiments, the wetting agent is lecithin.

In some embodiments, the chewable gel product contains one or more wetting agents in an amount that ranges from about 0.1% w/w to about 30% w/w. In some embodiments, the chewable gel product contains one or more wetting agents in an amount that ranges from about 1% w/w to about 25% w/w. In some embodiments, the chewable gel product contains one or more wetting agents in an amount that ranges from about 10% w/w to about 25% w/w. In some embodiments, the chewable gel product contains one or more wetting agents in an amount that ranges from about 14% w/w to about22% w/w. In some embodiments, the chewable gel product contains one or more wetting agents in an amount that ranges from about 1% w/w to about 10% w/w. In some embodiments, the chewable gel product contains one or more wetting agents in an amount that is less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, less than about 0.5% w/w, or less than about 0.1% w/w. In some embodiments, the chewable gel product contains one or more wetting agents in an amount that is greater than about 0.1% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, or greater than about 25% w/w. In some embodiments, the chewable gel product contains one or more wetting agents in an amount that has a range of % w/w having an upper limit of about 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, 1% w/w, or 0.5% w/w, and an independently selected lower limit of about 0.1% w/w, 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, or 25% w/w, where the lower limit is less than the upper limit. In some embodiments, the chewable gel product contains one or more wetting agents in an amount of at about 0.1% w/w, about 0.25% w/w, about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 11% w/w, about 12% w/w, about 13% w/w, about 14% w/w, about 14.5 % w/w, about 15% w/w, about 16% w/w, about 17% w/w, about 18% w/w, about 19% w/w, about 20% w/w, about 21% w/w, about 22% w/w, about 23% w/w, about 24% w/w, about 25% w/w, about 30% w/w, about 35% w/w, about 40% w/w, about 45% w/w, or about 30% w/w. In some embodiments, the chewable gel product contains the one or more wetting agents in an amount of about 1.80% w/w. In some embodiments, the chewable gel product contains the one or more wetting agents in an amount of about 1.50% w/w. It should be noted that the % w/w wetting agent values described herein may vary by ±0.2. For example 3% w/w wetting agent could vary from 2.8% w/w wetting agent to 3.2% w/w wetting agent.

### pH Adjusting Agents

In some aspects, the present disclosure relates to chewable gel products containing one or more non-acid-activated gel carriers (*e.g*., carrageenan, a combination of carrageenan and pectin, *etc.*) and one or more (*e.g.,* one or more, two or more, three or more, four or more, five or more, *etc.*) pH adjusting agents. In some embodiments, the pH adjusting agent may be any suitable acid, salts thereof, and derivatives thereof (*e.g.,* acid anhydride derivatives, *etc*.)*.* In some embodiments, the pH adjusting agent is one or more of citric acid, fumaric acid, malic acid, phosphoric acid, succinic acid, tartaric acid, maleic acid, acetic acid, phosphoric acid, hydrochloric acid, lactic acid, propionic acid, salts thereof, and derivatives thereof (*e.g*., acid anhydride derivatives, *etc*.)*.* In some embodiments, the pH adjusting agent is one or more of malic acid, phosphoric acid, and succinic acid, salts thereof, and derivatives thereof *(e.g.,* acid anhydride derivatives, *etc.).* In some embodiments, the pH adjusting agent is malic acid, salts thereof, or derivatives thereof *(e.g.,* acid anhydride derivatives, *etc.).* In some embodiments, the pH adjusting agent is phosphoric acid, salts thereof, or derivatives thereof *(e.g.,* acid anhydride derivatives, *etc.).* In some embodiments, the pH adjusting agent is succinic acid, salts thereof, or derivatives thereof *(e.g.,* acid anhydride derivatives, *etc.).*

In some embodiments, the pH value of the chewable gel product is measured prior to gelation, for example, while the product is still in a molten or liquid state. In some embodiments, the pH value may be measured prior to formulation with a non-acid-activated gel carrier. In some embodiments, the pH value may be measured after gelation of the chewable gel product.

In some embodiments, the present disclosure relates to methods for producing a chewable gel product by formulating a liquid composition having a gel carrier and an active pharmaceutical ingredient; adjusting the pH of the liquid composition to a pH that ranges from about 2.5 to about 8; and gelating the liquid composition at a temperature sufficient to yield a chewable gel product. In some embodiments, the gel carrier is a non-acid-activated gel carrier of the present disclosure. In some embodiments, the temperature sufficient to yield a chewable gel product includes heating the liquid composition having a non-acid-activated gel carrier and an active pharmaceutical ingredient to a temperature that ranges from about 100°C to about 125°C and then allowing the heated composition to gelate upon cooling. In some embodiments, the composition is heated to a temperature of about 100°C, about 105°C, about 110°C, about 115°C, about 120°C, or about 125°C. In some embodiments, the pH of the liquid composition having a non-acid-activated gel carrier is adjusted to a pH that ranges from about 3.9 to about 5. In some embodiments, the liquid composition having a non-acid-activated gel carrier has pH value that ranges from about 3.9 to about 5. In some embodiments, the gel carrier is an acid-activated gel carrier of the present disclosure. In some embodiments, the temperature sufficient to yield a chewable gel product includes heating the liquid composition having an acid-activated gel carrier and an active pharmaceutical ingredient to a temperature that ranges from about 45°C to about 90°C and then allowing the heated composition to gelate upon cooling. In some embodiments, the composition is heated to a temperature of about 45°C, about 50°C, about 55°C, about 60°C, about 65°C, about 70°C, about 75°C, about 80°C, about 85°C, or about 90°C. In some embodiments, the pH of the liquid composition having an acid-activated gel carrier is adjusted to a pH that ranges from about 4 to about 8. In some embodiments, the liquid composition having an acid-activated gel carrier has a pH value that ranges from about 4 to about 8. In some embodiments, the method further includes adding a taste-compatible acid of the present disclosure to the liquid composition in an amount that yields a pH value that ranges from 2.5 to 4.0.

In some embodiments, the pH value may be measured at a temperature that ranges from about 25°C to about 60°C. In some embodiments, the pH value may be measured at a temperature of about 25°C, about 26°C, about 27°C, about 28°C, about 29°C, about 30°C, about 31°C, about 32°C, about 33°C, about 34°C, about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, or about 60°C. Any known suitable instrument for measuring pH at various temperatures may be used. In some embodiments, the instrument for measuring pH has a temperature compensation feature. It is to be understood that different instruments for measuring pH may have variability in how the pH is measured and may thus yield different results. Also, an instrument of the present disclosure for measuring pH may yield a different result if not properly calibrated.

In some embodiments, the chewable gel product containing one or more non-acid-activated gel carriers has a pH value that ranges from about 3 to about 8. In some embodiments, the chewable gel product containing one or more non-acid-activated gel carriers has a pH value that ranges from about 3 to about 8, from about 3 to about 7.5, from about 3 to about 7, from about 3 to about 6.5, from about 3 to about 6, from about 3 to about 5.5, from about 3 to about 5, from about 3 to about 4.5, from about 3 to about 4, from about 3 to about 3.5, from about 3.5 to about 8, from about 3.5 to about 7.5, from about 3.5 to about 7, from about 3.5 to about 6.5, from about 3.5 to about 6, from about 3.5 to about 5.5, from about 3.5 to about 5, from about 3.9 to about 5, from about 3.5 to about 4.5, from about 3.5 to about 4, from about 4 to about 8, from about 4 to about 7.5, from about 4 to about 7, from about 4 to about 6.5, from about 4 to about 6, from about 4 to about 5.5, from about 4 to about 5, from about 4 to about 4.5, from about 4.5 to about 8, from about 4.5 to about 7.5, from about 4.5 to about 7, from about 4.5 to about 6.5, from about 4.5 to about 6, from about 4.5 to about 5.5, from about 4.5 to about 5, from about 5 to about 8, from about 5 to about 7.5, from about 5 to about 7, from about 5 to about 6.5, from about 5 to about 6, from about 5 to about 5.5, from about 5.5 to about 8, from about 5.5 to about 7.5, from about 5.5 to about 7, from about 5.5 to about 6.5, from about 5.5 to about 6, from about 6 to about 8, from about 6 to about 7.5, from about 6 to about 7, from about 6 to about 6.5, from about 6.5 to about 8, from about 6.5 to about 7.5, from about 6.5 to about 7, from about 7 to about 8, from about 7 to about 7.5, or from about 7.5 to about 7.5. In some embodiments, the chewable gel product containing one or more non-acid-activated gel carriers has a pH value of about 3, about 3.1, about 3.2, about 3.3, about 3.4, about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, about 7, about 7.1, about 7.2, about 7.3, about 7.4, about 6.5, about 7.6, about 7.7, about 7.8, about 6.9, or about 8. It should be noted that the pH values described herein may vary by ±0.2. For example a pH value of about 4 could vary from pH 3.8 to pH 4.2.

In other aspects, the present disclosure relates to chewable gel products containing one or more acid-activated gel carriers *(e.g.,* pectin and gelatin) and one or more *(e.g.,* one or more, two or more, three or more, four or more, five or more, *etc.)* taste-compatible pH adjusting agents. As used herein, the term "*taste-compatible pH adjusting agents"* refers to a pH adjusting agent that, when formulated with an API in a chewable gel product, does not give rise to substantial and/or detectable levels of bitterness of unpalatable taste in the chewable gel product. In some embodiments, a taste-compatible pH adjusting agent of the present disclosure is a taste-compatible acid, salt thereof, or derivative thereof (*e.g*., acid anhydride derivatives, *etc*.)*.* In some embodiments, the taste-compatible pH adjusting agent is one or more of fumaric acid, malic acid, phosphoric acid, succinic acid, tartaric acid, maleic acid, acetic acid, phosphoric acid, hydrochloric acid, lactic acid, propionic acid, salt thereof, and derivative thereof *(e.g.,* acid anhydride derivatives, *etc.).* In some embodiments, the taste-compatible pH adjusting agent is fumaric acid, a salt thereof, or a derivative thereof. In some embodiments, the taste-compatible pH adjusting agent is malic acid, a salt thereof, or a derivative thereof. In some embodiments, the taste-compatible pH adjusting agent is phosphoric acid, a salt thereof, or a derivative thereof. In some embodiments, the taste-compatible pH adjusting agent is succinic acid, a salt thereof, or a derivative thereof. In some embodiments, the taste-compatible pH adjusting agent is tartaric acid, a salt thereof, or a derivative thereof. In some embodiments, the taste-compatible pH adjusting agent is maleic acid, a salt thereof, or a derivative thereof. In some embodiments, the taste-compatible pH adjusting agent is acetic acid, a salt thereof, or a derivative thereof. In some embodiments, the taste-compatible pH adjusting agent is hydrochloric acid, a salt thereof, or a derivative thereof. In some embodiments, the taste-compatible pH adjusting agent is lactic acid, a salt thereof, or a derivative thereof. In some embodiments, the taste-compatible pH adjusting agent is propionic acid, a salt thereof, or a derivative thereof. In some embodiments, the taste compatible pH adjusting agent is not sodium citrate, citric acid, sodium ascorbate, or ascorbic acid. In some embodiments, the pH value of the chewable gel product is measured prior to gelation, for example, while the product is still in a molten or liquid state. In some embodiments, the pH value may be measured prior to formulation with an acid-activated gel carrier. In some embodiments, the pH value may be measured after gelation of the chewable gel product. In some embodiments, the pH value may be measured at a temperature that ranges from about 25°C to about 60°C. In some embodiments, the pH value may be measured at a temperature of about 25°C, about 26°C, about 27°C, about 28°C, about 29°C, about 30°C, about 31°C, about 32°C, about 33°C, about 34°C, about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, or about 60°C.

In some embodiments, the chewable gel product containing one or more acid-activated gel carriers contains one or more taste compatible pH adjusting agents in an amount that yields a pH value that ranges from about 2.4 to about 5.5. In some embodiments, the chewable gel product containing one or more acid-activated gel carriers contains one or more taste compatible pH adjusting agents in an amount that yields a pH value that ranges from about 2.4 to about 5.5, from about 2.4 to about 5, from about 2.4 to about 4.5, from about 2.4 to about 4, from about 2.4 to about 3.5, from about 2.4 to about 3.4, from about 2.4 to about 3.3, from about 2.4 to about 3.2, from about 2.4 to about 3.1, from about 2.4 to about 3, from about 2.4 to about 2.9, from about 2.4 to about 2.8, from about 2.4 to about 2.7, from about 2.4 to about 2.6, from about 2.4 to about 2.5, from about 2.5 to about 5.5, from about 2.5 to about 5, from about 2.5 to about 4.5, from about 2.5 to about 4, from about 2.5 to about 3.5, from about 2.5 to about 3.4, from about 2.5 to about 3.3, from about 2.5 to about 3.2, from about 2.5 to about 3.1, from about 2.5 to about 3, from about 2.5 to about 2.9, from about 2.5 to about 2.6, from about 2.6 to about 5.5, from about 2.6 to about 5, from about 2.6 to about 4.5, from about 2.6 to about 4, from about 2.6 to about 3.5, from about 2.6 to about 3.4, from about 2.6 to about 3.3, from about 2.6 to about 3.2, from about 2.6 to about 3.1, from about 2.6 to about 3, from about 2.6 to about 2.9, from about 2.6 to about 2.7, from about 2.7 to about 5.5, from about 2.7 to about 5, from about 2.7 to about 4.5, from about 2.7 to about 4, from about 2.7 to about 3.5, from about 2.7 to about 3.4, from about 2.7 to about 3.3, from about 2.7 to about 3.2, from about 2.7 to about 3.1, from about 2.7 to about 3, from about 2.7 to about 2.9, from about 2.7 to about 2.8, from about 2.8 to about 5.5, from about 2.8 to about 5, from about 2.8 to about 4.5, from about 2.8 to about 4, from about 2.8 to about 3.5, from about 2.8 to about 3.4, from about 2.8 to about 3.3, from about 2.8 to about 3.2, from about 2.8 to about 3.1, from about 2.8 to about 3, from about 2.8 to about 2.9, from about 2.9 to about 5.5, from about 2.9 to about 5, from about 2.9 to about 4.5, from about 2.9 to about 4, from about 2.9 to about 3.5, from about 2.9 to about 3.4, from about 2.9 to about 3.3, from about 2.9 to about 3.2, from about 2.9 to about 3.1, from about 2.9 to about 3, from about 3 to about 5.5, from about 3 to about 5, from about 3 to about 4.5, from about 3 to about 4, from about 3 to about 3.5, from about 3 to about 3.4, from about 3 to about 3.3, from about 3 to about 3.2, from about 3 to about 3.1, from about 3.1 to about 5.5, from about 3.1 to about 5, from about 3.1 to about 4.5, from about 3.1 to about 4, from about 3.1 to about 3.5, from about 3.1 to about 3.4, from about 3.1 to about 3.3, from about 3.1 to about 3.2, from about 3.2 to about 5.5, from about 3.2 to about 5, from about 3.2 to about 4.5, from about 3.2 to about 4, from about 3.2 to about 3.5, from about 3.2 to about 3.4, from about 3.2 to about 3.3, from about 3.3 to about 5.5, from about 3.3 to about 5, from about 3.3 to about 4.5, from about 3.3 to about 4, from about 3.3 to about 3.5, from about 3.3 to about 3.4, from about 3.4 to about 5.5, from about 3.4 to about 5, from about 3.4 to about 4.5, from about 3.4 to about 4, from about 3.4 to about 3.5, from about 3.5 to about 5.5, from about 3.5 to about 5, from about 3.5 to about 4.5, from about 3.5 to about 4, from about 4 to about 5.5, from about 4 to about 5, from about 4 to about 4.5, from about 4.5 to about 5.5, from about 4.5 to about 5, or from about 5 to about 5.5. In some embodiments, the chewable gel product containing one or more acid-activated gel carriers contains one or more taste compatible pH adjusting agents in an amount that yields a pH value of about 2.4, about 2.5, about 2.6, about 2.7, about 2.8, about 2.9, about 3.0, about 3.1, about 3.2, about 3.3, about 3.4, about 3.5, about 4, about 4.5, about 5, or about 5.5. It should be noted that the pH values described herein may vary by ±0.2. For example a pH value of about 4 could vary from pH 3.8 to pH 4.2.

### Base Vehicles

In some aspects, the present disclosure relates to chewable gel products containing one or more (*e.g.,* one or more, two or more, three or more, four or more, five or more, *etc.)* base vehicles. In some embodiments, base vehicles of the present disclosure refer to agents that provide bulk or body to the chewable gel products of the present disclosure. The one or more base vehicles may be any suitable base vehicles, including, for example, maltitol, maltodextrin, maltitol syrup, corn syrup, xylitol, sucrose, dextrose, maltose, fructose, mannitol, erythritol, glycerin, isomalt, polydextrose, lactitol, lycasin, sorbitol, and any combinations thereof. It is to be understood that any of the base vehicles of the present disclosure may be provided in a solid form or in a solution form that included the solid dissolved into a solution. In some embodiments, the vehicle base is maltitol. In some embodiments, the vehicle base is maltodextrin. In some embodiments, the vehicle base is maltitol syrup. In some embodiments, the vehicle base is corn syrup. In some embodiments, the vehicle base is xylitol. In some embodiments, the vehicle base is sucrose. In some embodiments, the vehicle base is dextrose. In some embodiments, the vehicle base is maltose. In some embodiments, the vehicle base is fructose. In some embodiments, the vehicle base is mannitol. In some embodiments, the vehicle base is erythritol. In some embodiments, the vehicle base is glycerin. In some embodiments, the vehicle base is isomalt. In some embodiments, the vehicle base is polydextrose. In some embodiments, the vehicle base is lactitol. In some embodiments, the vehicle base is lycasin.

In some embodiments, the chewable gel product contains one or more base vehicles in an amount that ranges from about 0.5% w/w to about 90% w/w. In some embodiments, the chewable gel product contains one or more base vehicles in an amount that ranges from about 10% w/w to about 60% w/w. In some embodiments, the chewable gel product contains one or more base vehicles in an amount that ranges from about 45% w/w to about 60% w/w. In some embodiments, the chewable gel product contains one or more base vehicles in an amount that is less than about 90% w/w, less than about 80% w/w, less than about 70% w/w, less than about 60% w/w, less than about 50% w/w, less than about 40% w/w, less than about 30% w/w, less than about 25% w/w, less than about 20% w/w, less than about 15% w/w, less than about 10% w/w, less than about 9% w/w, less than about 8% w/w, less than about 7% w/w, less than about 6% w/w, less than about 5% w/w, less than about 4% w/w, less than about 3% w/w, less than about 2% w/w, less than about 1% w/w, or less than about 0.5% w/w. In some embodiments, the chewable gel product contains one or more base vehicles in an amount that is greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 15% w/w, greater than about 20% w/w, greater than about 25% w/w, greater than about 30% w/w, greater than about 40% w/w, greater than about 50% w/w, greater than about 60% w/w, greater than about 70% w/w, greater than about 80% w/w, or greater than about 90% w/w. In some embodiments, the chewable gel product contains one or more base vehicles in an amount that has a range of % w/w having an upper limit of about 90% w/w, about 80% w/w, about 70% w/w, about 60% w/w, 50% w/w, 40% w/w, 30% w/w, 25% w/w, 20% w/w, 15% w/w, 10% w/w, 9% w/w, 8% w/w, 7% w/w, 6% w/w, 5% w/w, 4% w/w, 3% w/w, 2% w/w, or 1% w/w, and an independently selected lower limit of about 0.5% w/w, 1% w/w, 2% w/w, 3% w/w, 4% w/w, 5% w/w, 6% w/w, 7% w/w, 8% w/w, 9% w/w, 10% w/w, 15% w/w, 20% w/w, 25% w/w, 30% w/w, 40% w/w, 50% w/w, 60% w/w, 70% w/w, 80% w/w, or 90% w/w, where the lower limit is less than the upper limit. In some embodiments, the chewable gel product contains one or more base vehicles in an amount of at about 0.5% w/w, about 0.75% w/w, about 1% w/w, about 2% w/w, about 3% w/w, about 4% w/w, about 5% w/w, about 6% w/w, about 7% w/w, about 8% w/w, about 9% w/w, about 10% w/w, about 15% w/w, about 20% w/w, about 25% w/w, about 30% w/w, a about 40% w/w, about 50% w/w, about 51% w/w, about 52% w/w, about 53% w/w, about 54% w/w, about 55% w/w, about 56% w/w, about 57% w/w, about 58% w/w, about 59% w/w, about 60% w/w, about 70% w/w, about 80% w/w, or about 90% w/w. It should be noted that the % w/w base vehicle values described herein may vary by ±0.20. For example 50% w/w base vehicle could vary from 49.8% w/w base vehicle to 50.2% w/w base vehicle.

### Sweeteners

In some aspects, the present disclosure relates to chewable gel products containing one or more *(e.g.,* one or more, two or more, three or more, four or more, five or more, *etc.)* sweeteners. In some embodiments, sweeteners of the present disclosure may be natural sweeteners *(e.g.,* sucrose, trehalose, inulin *etc.)* or artificial sweeteners *(e.g.,* sucralose, saccharin, aspartame, *etc.).* Examples of suitable sweeteners, include, for example, sucrose, aspartame, sucralose, saccharin, sodium saccharin, acesulfame potassium, stevia, sodium cyclamate, inulin, isomalt, maltose, neohesperidin dihydrochalcone, trehalose, thaumatin, sorbitol, maltitol, and any combination thereof. In some embodiments, the sweetener is sucrose. In some embodiments, the sweetener is aspartame. In some embodiments, the sweetener is sucralose. In some embodiments, the sweetener is saccharin. In some embodiments, the sweetener is acesulfame potassium. In some embodiments, the sweetener is stevia. In some embodiments, the sweetener is sodium cyclamate. In some embodiments, the sweetener is inulin. In some embodiments, the sweetener is isomalt. In some embodiments, the sweetener is maltose. In some embodiments, the sweetener is neohesperidin dihydrochalcone. In some embodiments, the sweetener is trehalose. In some embodiments, the sweetener is thaumatin. In some embodiments, the sweetener is sorbitol. In some embodiments, the sweetener is maltitol. In some embodiments, the sweetener is a combination of a natural sweetener *(e.g.,* sucrose) and an artificial sweetener *(e.g.,* sucralose). In some embodiments, the sweetener is a combination of sorbitol, maltitol, aspartame, and sucralose. It is to be understood that any of the sweeteners of the present disclosure may be provided in a solid form or in a solution form that included the solid dissolved into a solution.

In some embodiments, the chewable gel product contains one or more sweeteners in an amount that ranges from about 0.01% w/w to about 5% w/w. In some embodiments, the chewable gel product contains one or more sweeteners in an amount that ranges from about 0. 10% w/w to about 1% w/w. In some embodiments, the chewable gel product contains one or more sweeteners in an amount that ranges from about 0.10% w/w to about 0.50% w/w. In some embodiments, the chewable gel product contains one or more sweeteners in an amount that is less than about 5% w/w, less than about 4.5% w/w, less than about 4% w/w, less than about 3.5% w/w, less than about 3% w/w, less than about 2.5% w/w, less than about 2% w/w, less than about 1.5% w/w, less than about 1% w/w, less than about 0.75% w/w, less than about 0.5% w/w, less than about 0.25% w/w, less than about 0.1% w/w, less than about 0.075% w/w, less than about 0.05% w/w, less than about 0.025% w/w, or less than about 0.01% w/w. In some embodiments, the chewable gel product contains one or more sweeteners in an amount that is greater than about 0.01% w/w, greater than about 0.025% w/w, greater than about 0.05% w/w, greater than about 0.075% w/w, greater than about 0.1% w/w, greater than about 0.25% w/w, greater than about 0.5% w/w, greater than about 0.75% w/w, greater than about 1% w/w, greater than about 1.5% w/w, greater than about 2% w/w, greater than about 2.5% w/w, greater than about 3% w/w, greater than about 3.5% w/w, greater than about 4% w/w, greater than about 4.5% w/w, or greater than about 5% w/w. In some embodiments, the chewable gel product contains one or more sweeteners in an amount that has a range of % w/w having an upper limit of about 5% w/w, 4.5% w/w, 4% w/w, 3.5% w/w, 3% w/w, 2.5% w/w, 2% w/w, 1.5% w/w,1% w/w, 0.75% w/w, 0.5% w/w, 0.25% w/w, 0.1%, 0.075% w/w, 0.05% w/w, 0.025% w/w, or 0.01% w/w, and an independently selected lower limit of about 0.01% w/w, 0.025% w/w, 0.05% w/w, 0.075% w/w, 0.1% w/w, 0.25% w/w,0.5% w/w, 0.75% w/w, 1% w/w, 1.5% w/w, 2% w/w, 2.5% w/w, 3% w/w, 3.5% w/w, 4% w/w, 4.5%, or w/w,5% w/w, where the lower limit is less than the upper limit. In some embodiments, the chewable gel product contains one or more sweeteners in an amount of at about 0.01% w/w, about 0.025% w/w, about 0.05% w/w, about 0.075% w/w, about 0.1% w/w, about 0.20% w/w, about 0.25% w/w, about 0.5% w/w, about 0.75% w/w, about 1% w/w, about 1.5% w/w, about 2% w/w, about 2.5% w/w, about 3% w/w, about 3.5% w/w, about 4% w/w, about 4.5% w/w, or about 5%. It should be noted that the %w/w sweetener values described herein may vary by ±0.02. For example 0.20% w/w sweetener could vary from 0.198%w/w sweetener to 0.220% w/w sweetener.

### Flavorants

In some aspects, the present disclosure relates to chewable gel products containing one or more *(e.g.,* one or more, two or more, three or more, four or more, five or more, *etc.)* flavorants. The one or more flavorant may be any suitable flavorant. The one or more flavorants may contain any suitable flavor, including, for example, a mixed berry flavor, a mulberry flavor, a cherry flavor, a strawberry flavor, a citrus flavor, a lemon flavor, a lime flavor, an orange flavor, a grape flavor, a vanilla flavor, a chocolate flavor, a caramel flavor, a mint flavor, a spearmint flavor, a wintergreen flavor, a menthol flavor, and any combinations thereof.

In some embodiments, the chewable gel product contains the one or more flavorants in an amount that ranges from about 0.05% w/w to about 5% w/w. In some embodiments, the chewable gel product contains the one or more flavorants in an amount that ranges from about 0.10% w/w to about 1% w/w. In some embodiments, the chewable gel product contains the one or more flavorants in an amount that ranges from about 0.10% w/w to about 0.50% w/w. In some embodiments, the chewable gel product contains the one or more flavorants in an amount that is less than about 5% w/w, less than about 4% w/w, less than about 3% w/w, less than about 2% w/w, less than about 1.75% w/w, less than about 1.5% w/w, less than about 1.25% w/w, less than about 1% w/w, less than about 0.75% w/w, less than about 0.5% w/w, less than about 0.25% w/w, less than about 0.1% w/w, less than about 0.075% w/w, or less than about 0.05% w/w. In some embodiments, the chewable gel product contains the one or more flavorants in an amount that is greater than about 0.05% w/w, greater than about 0.075% w/w, greater than about 0.1% w/w, greater than about 0.25% w/w, greater than about 0.5% w/w, greater than about 0.75% w/w, greater than about 1% w/w, greater than about 1.25% w/w, greater than about 1.5% w/w, greater than about 1.75% w/w, greater than about 2% w/w, greater than about 3% w/w, greater than about 4% w/w, greater than about 4.25% w/w, greater than about 4.5% w/w, or greater than about 4.75% w/w,. In some embodiments, the chewable gel product contains the one or more flavorants in an amount that has a range of % w/w having an upper limit of 5% w/w, 4% w/w, 3% w/w, 2% w/w, 1.75% w/w, 1.5% w/w, 1.25% w/w, 1% w/w, 0.75% w/w, 0.5% w/w, 0.25% w/w, 0.1% w/w, or 0.075% w/w, and an independently selected lower limit of 0.05% w/w, 0.075% w/w, 0.1% w/w, 0.25% w/w, 0.5% w/w, 0.75% w/w, 1% w/w, 1.25% w/w, 1.5% w/w, 1.75% w/w, 2% w/w, 3% w/w, 4% w/w, 4.25% w/w, 4.5% w/w, or 4.75% w/w, where the lower limit is less than the upper limit. In some embodiments, the chewable gel product contains the one or more flavorants in an amount of about 0.05% w/w, about 0.075% w/w, about 0.10% w/w, about 0.20% w/w, about 0.25% w/w, about 0.30% w/w, about 0.40% w/w, about 0.50% w/w, about 0.60% w/w, about 0.70% w/w, about 0.75% w/w, about 0.80% w/w, about 0.90% w/w, about 1% w/w, about 1.1% w/w, about 1.2% w/w, about 1.25% w/w, about 1.3% w/w, about 1.4% w/w, about 1.5% w/w, about 1.6% w/w, about 1.7% w/w, about 1.75% w/w, about 1.8% w/w, about 1.9% w/w, about 2% w/w, about 2.25% w/w, about 2.5% w/w, about 2.75% w/w, about 3% w/w, about 3.25% w/w, about 3.5% w/w, about 3.75% w/w, about 4% w/w, about 4.25% w/w, about 4.5% w/w, about 4.75% w/w, or about 5% w/w, . In some embodiments, the chewable gel product contains the one or more flavorants at about 0.25% w/w. In some embodiments, the chewable gel product contains the one or more flavorants in an amount of about 0.4% w/w. In some embodiments, the chewable gel product contains the one or more flavorants in an amount of about 0.8% w/w. It should be noted that the %w/w flavorant values described herein may vary by ±0.02. For example 0.20% w/w flavorant could vary from 0.198%w/w flavorant to 0.220% w/w flavorant.

### Colorants

In some aspects, the present disclosure relates to chewable gel products containing one or more *(e.g.,* one or more, two or more, three or more, four or more, five or more, *etc.)* colorants. The chewable gel products may be any suitable color. In some embodiments, colorants of the present disclosure may be natural colorants or artificial colorants. Examples of suitable colorants may include, for example, FD&C Red 40, D&C Reds 3, 22, 28, 33 and 36, FD&C Yellows 5 and 6, D&C Yellow 10, FD&C Blues 1 and 2, FD&C Green 3, red iron oxide, caramel, beta-carotene, carmine, and any combinations thereof.

In some embodiments, the chewable gel product contains the one or more colorants in an amount that ranges from about 0.001% w/w to about 0.3% w/w. In some embodiments, the chewable gel product contains the one or more colorants in an amount that ranges from about 0.010% w/w to about 0.10% w/w. In some embodiments, the chewable gel product contains the one or more colorants in an amount that is less than about 0.3% w/w, less than about 0.25% w/w, less than about 0.2% w/w, less than about 0.1% w/w, less than about 0.075% w/w, less than about 0.05% w/w, less than about 0.025% w/w, less than about 0.01% w/w, less than about 0.0075% w/w, less than about 0.005% w/w, or less than about 0.0025% w/w. In some embodiments, the chewable gel product contains the one or more colorants in an amount that is greater than about 0.001% w/w, greater than about 0.0025% w/w, greater than about 0.005% w/w, greater than about 0.0075% w/w, greater than about 0.01% w/w, greater than about 0.025% w/w, greater than about 0.05% w/w, greater than about 0.075% w/w, greater than about 0.1% w/w, greater than about 0.2% w/w, or greater than about 0.25% w/w. In some embodiments, the chewable gel product contains the one or more colorants in an amount that has a range of % w/w having an upper limit of 0.3% w/w, 0.25% w/w, 0.2% w/w, 0.1% w/w, 0.075% w/w, 0.05% w/w, 0.025% w/w, 0.01% w/w, 0.0075% w/w, 0.005% w/w, or 0.0025% w/w, and an independently selected lower limit of 0.001% w/w, 0.0025% w/w, 0.005% w/w, 0.0075% w/w, 0.01% w/w, 0.025% w/w, 0.05% w/w, 0.075% w/w, 0.1% w/w, 0.2% w/w, 0.25% w/w, or 0.3% w/w, where the lower limit is less than the upper limit. In some embodiments, the chewable gel product contains the one or more colorants in an amount of about 0.001% w/w, about 0.0025% w/w, about 0.005% w/w, about 0.0075% w/w, about 0.01% w/w, about 0.015% w/w, about 0.02% w/w, about 0.025% w/w, about 0.03% w/w, about 0.035% w/w, about 0.04% w/w, about 0.045% w/w, about 0.05% w/w, about 0.055% w/w, about 0.06% w/w, about 0.065% w/w, about 0.07% w/w, about 0.075% w/w, about 0.08% w/w, about 0.085% w/w, about 0.09% w/w, about 0.1% w/w, about 0.2% w/w, 0.25% w/w, or about 0.3% w/w. In some embodiments, the chewable gel product contains the one or more colorants in an amount of about 0.015% w/w. It should be noted that the %w/w colorant values described herein may vary by ±0.002. For example 0.015% w/w colorant could vary from 0.013%w/w colorant to 0.017% w/w colorant.

### Water Content

In some aspects, the present disclosure relates to a chewable gel product containing water content in an amount that ranges from about 5% w/w to about 25% w/w of the final formulation of a chewable gel product of the present disclosure. In some embodiments, the chewable gel product contains water in an amount that ranges from about 5% w/w to about 25% w/w. In some embodiments, the chewable gel product contains water in an amount that ranges from about 15% w/w to about 25% w/w. In some aspects, the present disclosure relates to a chewable gel product containing water content in an amount that ranges from about 5% w/w to about 25% w/w, from about 5% w/w to about 22.5% w/w, from about 5% w/w to about 20% w/w, from about 5% w/w to about 17.5% w/w, from about 5% w/w to about 15% w/w, from about 5% w/w to about 12.5% w/w, from about 5% w/w to about 10% w/w, from about 5% w/w to about 7.5% w/w, from about 7.5% w/w to about 25% w/w, from about 7.5% w/w to about 22.5% w/w, from about 7.5% w/w to about 20% w/w, from about 7.5% w/w to about 17.5% w/w, from about 7.5% w/w to about 15% w/w, from about 7.5% w/w to about 12.5% w/w, from about 7.5% w/w to about 10% w/w, from about 10% w/w to about 7.5% w/w, from about 10% w/w to about 25% w/w, from about 10% w/w to about 22.5% w/w, from about 10% w/w to about 20% w/w, from about 10% w/w to about 17.5% w/w, from about 10% w/w to about 15% w/w, from about 10% w/w to about 12.5% w/w, from about 12.5% w/w to about 25% w/w, from about 12.5% w/w to about 22.5% w/w, from about 12.5% w/w to about 20% w/w, from about 12.5% w/w to about 17.5% w/w, from about 12.5% w/w to about 15% w/w, from about 15% w/w to about 25% w/w, from about 15% w/w to about 22.5% w/w, from about 15% w/w to about 20% w/w, from about 15% w/w to about 17.5% w/w, from about 17.5% w/w to about 25% w/w, from about 17.5% w/w to about 22.5% w/w, from about 17.5% w/w to about 20% w/w, from about 20% w/w to about 25% w/w, from about 20% w/w to about 22.5% w/w, or from about 22.5% w/w to about 25% w/w of the final formulation of a chewable gel product of the present disclosure. In some embodiments, the chewable gel product contains water content in an amount of about 5% w/w, 7.5% w/w, 10% w/w, 12.5% w/w, 15% w/w, 17.5% w/w, 20% w/w, 20.5% w/w, 21% w/w, 21.5% w/w, 22% w/w, 22.5% w/w, or 25% w/w of the final formulation of a chewable gel product of the present disclosure. It should be noted that the %w/w water values described herein may vary by ±0.2. For example 21% w/w water could vary from 20.8%w/w water to 21.2% w/w water.

### Chewable Bite Index

In some aspects, the present disclosure relates to chewable gel products formulated to provide a mouthfeel chewiness texture that ranges from a leathery or rubbery texture to a clean bite texture. In some embodiments, the desired mouthfeel chewiness texture of a chewable gel product of the present disclosure may be achieved by the choice of gel carrier(s), pH adjusting agent(s), wetting agent(s), base vehicle(s), and any combinations thereof. In some embodiments, the mouthfeel chewiness texture of the chewable gel product may be measured by a chewable bite index (CBI).

In some embodiments, the chewable bite index (CBI) of a chewable gel product of the present disclosure is assigned by a panel of tasters. The CBI may be calculated by each member of the panel assigning a numerical value to the chewable gel product based on the mouthfeel chewiness of the chewable gel product, where the numerical value is a measure of degree of chewiness scaled from 1 (extremely leathery or rubbery texture) to 5 (clean bite texture), and the CBI is calculated by averaging the scores from each member on the panel to identify the CBI of the given chewable gel product.

In some embodiments, a CBI score of about 1 to about 2 indicates a chewable gel product that has a very chewy mouthfeel texture. In some embodiments, a CBI score of about 3 to about 4 indicates a chewable gel product that has a slightly chewy and a more clean bite mouthfeel texture. In some embodiments, a CBI score of about 5 indicates a chewable gel product that has very clean bite mouthfeel texture.

In some embodiments, the chewable gel product may be formulated to yield an API-containing composition having a chewable bite index (CBI) of about 1 to about 5. In some embodiments, the chewable gel product may be formulated to yield an API-containing composition having a chewable bite index (CBI) of about 1 to about 5, about 1 to about 4, about 1 to about 3, about 1 to about 2, about 2 to about 5, about 2 to about 4, about 2 to about 3, about 3 to about 5, about 3 to about 4, or about 4 to about 5. In some embodiments, the chewable gel product has a CBI of greater than about 1, greater than about 1.1, greater than about 1.2, greater than about 1.3, greater than about 1.4, greater than about 1.5, greater than about 1.6, greater than about 1.7, greater than about 1.8, greater than about 1.9, greater than about 2, greater than about 2.1, greater than about 2.2, greater than about 2.3, greater than about 2.4, greater than about 2.5, greater than about 2.6, greater than about 2.7, greater than about 2.8, greater than about 2.9,greater than about 3, greater than about 3.1, greater than about 3.2, greater than about 3.3, greater than about 3.4, greater than about 3.5, greater than about 3.6, greater than about 3.7, greater than about 3.8, greater than about 3.9, greater than about 4, greater than about 4.1, greater than about 4.2, greater than about 4.3, greater than about 4.4, greater than about 4.5, greater than about 4.6, greater than about 4.7, greater than about 4.8, greater than about 4.9, or greater than about 5. In some embodiments, the chewable gel product has a CBI of about 1, about 1.1, about 1.2, about 1.3, about 1.4, about 1.5, about 1.6, about 1.7, about 1.8, about 1.9, about 2, about 2.1, about 2.2, about 2.3, about 2.4, about 2.5, about 2.6, about 2.7, about 2.8, about 2.9,about 3, about 3.1, about 3.2, about 3.3, about 3.4, about 3.5, about 3.6, about 3.7, about 3.8, about 3.9, about 4, about 4.1, about 4.2, about 4.3, about 4.4, about 4.5, about 4.6, about 4.7, about 4.8, about 4.9, or about 5.

### Shapes and Sizes

Chewable gel products of the present disclosure may have any suitable shape, including, for example, spheres, ovals, cylinders, rectangular boxes, cubes, fruit shapes (*e.g*., the shape of berries, citrus fruits, segments of citrus fruits, *etc.),* plant shapes, animal shapes, worms, and any combinations thereof.

In some embodiments, a chewable gel product of the present disclosure has a total weight of from about 100 mg to about 10,000 mg. In some embodiments, the chewable gel product has a total weight of from about 100 mg to about 10,000 mg, from about 100 mg to about 7,500 mg, from about 100 mg to about 5,000 mg, from about 100 mg to about 4,000 mg, from about 100 mg to about 3,000 mg, from about 100 mg to about 2,500 mg, from about 100 mg to about 2,000 mg, from about 100 mg to about 1,500 mg, from about 100 mg to about 1,000 mg, from about 100 mg to about 750 mg, from about 100 mg to about 500 mg, from about 100 mg to about 250 mg, from about 250 mg to about 10,000 mg, from about 250 mg to about 7,500 mg, from about 250 mg to about 5,000 mg, from about 250 mg to about 4,000 mg, from about 250 mg to about 3,000 mg, from about 250 mg to about 2,500 mg, from about 250 mg to about 2,000 mg, from about 250 mg to about 1,500 mg, from about 250 mg to about 1,000 mg, from about 250 mg to about 750 mg, from about 250 mg to about 500 mg, from about 500 mg to about 10,000 mg, from about 500 mg to about 7,500 mg, from about 500 mg to about 5,000 mg, from about 500 mg to about 4,000 mg, from about 500 mg to about 3,000 mg, from about 500 mg to about 2,500 mg, from about 500 mg to about 2,000 mg, from about 500 mg to about 1,500 mg, from about 500 mg to about 1,000 mg, from about 500 mg to about 750 mg, from about 750 mg to about 10,000 mg, from about 750 mg to about 7,500 mg, from about 750 mg to about 5,000 mg, from about 750 mg to about 4,000 mg, from about 750 mg to about 3,000 mg, from about 750 mg to about 2,500 mg, from about 750 mg to about 2,000 mg, from about 750 mg to about 1,500 mg, from about 750 mg to about 1,000 mg, from about 1,000 mg to about 10,000 mg, from about 1,000 mg to about 7,500 mg, from about 1,000 mg to about 5,000 mg, from about 1,000 mg to about 4,000 mg, from about 1,000 mg to about 3,000 mg, from about 1,000 mg to about 2,500 mg, from about 1,000 mg to about 2,000 mg, from about 2,000 mg to about 10,000 mg, from about 2,000 mg to about 7,500 mg, from about 2,000 mg to about 5,000 mg, from about 2,000 mg to about 4,000 mg, from about 2,000 mg to about 3,,000 mg, from about 2,000 mg to about 2,500 mg, from about 2,500 mg to about 10,000 mg, from about 2,500 mg to about 7,500 mg, from about 2,500 mg to about 5,000 mg, from about 2,500 mg to about 4,000 mg, from about 2,500 mg to about 3,000 mg, from about 3,000 mg to about 10,000 mg, from about 3,000 mg to about 7,500 mg, from about 3,000 mg to about 5,000 mg, from about 3,000 mg to about 4,000 mg, from about 4,000 mg to about 10,000 mg, from about 4,000 mg to about 7,500 mg, from about 4,000 mg to about 5,000 mg, from about 5,000 mg to about 10,000 mg, from about 5,000 mg to about 7,500 mg, or from about 7,500 mg to about 10,000 mg. In some embodiments, the chewable gel product has a total weight of about 2,000 mg.

### Formulated Chewable Gels

In some embodiments, a chewable gel product of the present disclosure contains from about 0.5% w/w to about 5% w/w non-acid-activated gel carrier containing carrageenan, and from about 0.01% w/w to about 10% w/w loratadine. In some embodiments, the chewable gel product contains from about 0.75% w/w to about 5% w/w non-acid-activated gel carrier containing carrageenan. In some embodiments, the chewable gel product contains from about 1% w/w to about 3% w/w non-acid-activated gel carrier containing carrageenan. In some embodiments, the chewable gel product contains about 1.8% w/w non-acid-activated gel carrier containing carrageenan. In some embodiments, the chewable gel product contains from about 0.05% w/w to about 5% w/w loratadine. In some embodiments, the chewable gel product contains from about 0. 1% w/w to about 3% w/w loratadine. In some embodiments, the chewable gel product contains about 0. 5% w/w loratadine. In some embodiments, the chewable gel product further contains a wetting agent, a sweetener, a colorant, a flavorant, a base vehicle, and water. In some embodiments, the taste acceptability index (TAI) is from about 6 to about 10. In some embodiments, the chewable gel product has a pH value that ranges from about 3.9 to about 5. In some embodiments, the pH value of the chewable gel product is measured prior to gelation, for example, while the product is still in a molten or liquid state. In some embodiments, the pH value may be measured prior to formulation with the non-acid-activated gel carrier containing carrageenan. In some embodiments, the chewable gel product further includes one or more base vehicles. In some embodiments, the gel product further includes sorbitol. In some embodiments, the chewable gel product contains sorbitol in an amount that ranges from about 10% w/w to about 60% w/w. In some embodiments, the chewable gel product contains sorbitol in an amount that ranges from about 45% w/w to about 60% w/w. In some embodiments, the chewable gel product contains about 51 % w/w of sorbitol. In some embodiments, the chewable gel product contains about 58 % w/w of sorbitol. In some embodiments, the chewable gel product further includes one or more of maltitol and propylene glycol. In some embodiments, the chewable gel product further includes maltitol and propylene glycol. In some embodiments, the chewable gel product contains each of the maltitol and propylene glycol in an amount that ranges from about 1% w/w to about 25% w/w. In some embodiments, the chewable gel product contains maltitol in an amount that ranges from about 14% w/w to about 22% w/w. In some embodiments, the chewable gel product contains about 14.5 % w/w of maltitol. In some embodiments, the chewable gel product contains about 22 % w/w of maltitol. In some embodiments, the chewable gel product contains propylene glycol in an amount that ranges from about 1% w/w to about 10% w/w. In some embodiments, the chewable gel product contains about 3 % w/w of propylene glycol. In some embodiments, the chewable gel product further includes one or more sweeteners. In some embodiments, the one or more sweeteners are selected from aspartame, sucralose, and any combination thereof. In some embodiments, the one or more sweeteners are aspartame and sucralose. In some embodiments, the chewable gel product contains each of the one or more sweeteners in an amount that ranges from about 0.01% w/w to about 5% w/w. In some embodiments, the chewable gel product contains each of the one or more sweeteners in an amount that ranges from about 0.10% w/w to about 1% w/w. In some embodiments, the chewable gel product contains aspartame in an amount that ranges from about 0.10% w/w to about 0.50% w/w. In some embodiments, the chewable gel product contains about 0.20 % w/w of aspartame. In some embodiments, the chewable gel product contains sucralose n an amount that ranges from about 0.10% w/w to about 0.50% w/w. In some embodiments, the chewable gel product contains about 0.20 % w/w of sucralose. In some embodiments, the chewable gel product further includes water. In some embodiments, the chewable gel product contains water in an amount that ranges from about 5% w/w to about 25% w/w. In some embodiments, the chewable gel product contains water in an amount that ranges from about 15% w/w to about 25% w/w. In some embodiments, the chewable gel product contains about 21 % w/w of water. In some embodiments, the chewable gel product further includes one or more flavorants. In some embodiments, the chewable gel product further includes a flavorant. In some embodiments, the chewable gel product contains the flavorant in an amount that ranges from about 0.05% w/w to about 2% w/w. In some embodiments, the chewable gel product contains the flavorant in an amount that ranges from about 0.10% w/w to about 1% w/w. In some embodiments, the chewable gel product contains the flavorant in an amount that ranges from about 0.10% w/w to about 0.50% w/w. In some embodiments, the chewable gel product contains about 0.40 % w/w of flavorant. In some embodiments, the flavorant is a mulberry flavor. In some embodiments, the chewable gel product further includes one or more colorants. In some embodiments, the chewable gel product further includes a colorant. In some embodiments, the chewable gel product contains the colorant in an amount that ranges from about 0.001% w/w to about 0.3% w/w. In some embodiments, the chewable gel product contains the colorant in an amount that ranges from about 0.010% w/w to about 0.10% w/w.. In some embodiments, the chewable gel product contains about 0.015 % w/w of colorant. In some embodiments, the colorant is FD&C Red No. 40. In some embodiments, the chewable gel product further includes a coating. In some embodiments, the coating includes one or more of MCT coconut oil and carnauba wax. In some embodiments, the coating includes MCT coconut oil and carnauba wax. In some embodiments, the chewable gel product further includes a source of cations. In some embodiments, the source of cations is potassium citrate. In some embodiments, the chewable gel product contains potassium citrate in an amount that ranges from about 0.10% w/w to about 1% w/w. In some embodiments, the chewable gel product contains about 0.20 % w/w of potassium citrate.

In some embodiments, a chewable gel product of the present disclosure contains from about 0.5% w/w to about 10% w/w non-acid-activated gel carrier containing carrageenan and pectin, and from about 0.01% w/w to about 10% w/w loratadine. In some embodiments, the chewable gel product contains from about 0.75% w/w to about 5% w/w non-acid-activated gel carrier containing carrageenan and pectin. In some embodiments, the chewable gel product contains from about 1% w/w to about 3% w/w non-acid-activated gel carrier containing carrageenan and pectin. In some embodiments, the chewable gel product contains from about 0.05% w/w to about 5% w/w loratadine. In some embodiments, the chewable gel product contains from about 0. 1% w/w to about 3% w/w loratadine. In some embodiments, the chewable gel product further contains a wetting agent, a sweetener, a colorant, a flavorant, a base vehicle, and water. In some embodiments, the taste acceptability index (TAI) is 6 to 10. In some embodiments, the chewable gel product has a pH value that ranges from about 3.9 to about 5. In some embodiments, the pH value of the chewable gel product is measured prior to gelation, for example, while the product is still in a molten or liquid state. In some embodiments, the pH value may be measured prior to formulation with the non-acid-activated gel carrier containing carrageenan and pectin.

In some embodiments, a chewable gel product of the present disclosure contains from about 0.5% w/w to about 5% w/w pectin, from about 0.01% w/w to about 10% w/w loratadine, and a pH adjusting agent. In some embodiments, the chewable gel product contains from about 0.75% w/w to about 4% w/w pectin. In some embodiments, the chewable gel product contains from about 1% w/w to about 3% w/w pectin. In some embodiments, the chewable gel product contains from about 0.05% w/w to about 5% w/w loratadine. In some embodiments, the chewable gel product contains from about 0. 1% w/w to about 3% w/w loratadine. In some embodiments, the chewable gel product further contains a wetting agent, a sweetener, and water. In some embodiments, the chewable gel product contains the pH adjusting agent in an amount sufficient to yield a pH value that ranges from about 2.6 to about 3.1. In some embodiments, the chewable gel product contains the pH adjusting agent in an amount sufficient to yield a taste acceptability index (TAI) of 6 to 10. In some embodiments, the pH value of the chewable gel product is measured prior to gelation, for example, while the product is still in a molten or liquid state. In some embodiments, the pH value may be measured prior to formulation with the pectin. In some embodiments, the pH value may be measured after gelation of the chewable gel product.

In some embodiments, a chewable gel product of the present disclosure contains from about 0.5% w/w to about 10% w/w gelatin, from about 0.01% w/w to about 10% w/w loratadine, and a pH adjusting agent. In some embodiments, the chewable gel product contains from about 0.5% w/w to about 7% w/w gelatin. In some embodiments, the chewable gel product contains from about 0.5% w/w to about 5% w/w gelatin. In some embodiments, the chewable gel product contains from about 0.05% w/w to about 5% w/w loratadine. In some embodiments, the chewable gel product contains from about 0. 1% w/w to about 3% w/w loratadine. In some embodiments, the chewable gel product further contains a wetting agent, a sweetener, and water. In some embodiments, the chewable gel product contains the pH adjusting agent in an amount sufficient to yield a pH value that ranges from about 2.6 to about 3.1. In some embodiments, the chewable gel product contains the pH adjusting agent in an amount sufficient to yield a taste acceptability index (TAI) of 6 to 10. In some embodiments, the pH value of the chewable gel product is measured prior to gelation, for example, while the product is still in a molten or liquid state. In some embodiments, the pH value may be measured prior to formulation with the gelatin. In some embodiments, the pH value may be measured after gelation of the chewable gel product.

### EXAMPLES

The present disclosure will be more fully understood by reference to the following examples. They should not, however, be construed as limiting the scope of the present disclosure. It is understood that the examples and embodiments described herein are for illustrative purposes only and that various modifications or changes in light thereof will be suggested to persons skilled in the art and are to be included within the spirit and purview of this application and scope of the appended claims.

### Example 1: Effects of different parameters on the Taste Acceptability Index

The following example describes the effects of various components on the levels of bitter and/or unpalatable taste profiles detected in chewable gel products containing active pharmaceutical ingredients.

### Materials and Methods

Taste Acceptability Index: The taste acceptability index for the indicated formulations was assessed by a panel of tasters tasting each formulation and assigning a TAI value as a measure of degree of bitterness scaled from 0-10. A score of 0 was assigned when a formulation had an extremely bitter and unpalatable taste profile, while a score of 10 was assigned when a formulation lacked any detectable bitterness. Intermediate scores were assigned by members on the panel based on the degree of bitterness. Water and crackers were administered between each round of tasting to neutralize (cleanse) the palate of each taster.

### Results

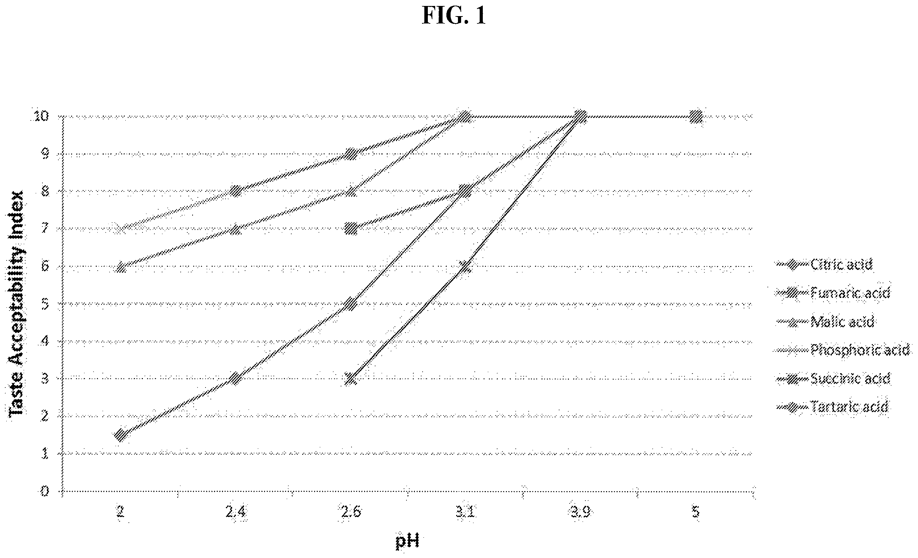

To begin developing chewable gel products containing active pharmaceutical agents that had desirable properties (*e.g*., appropriate texture/mouthfeel, a pleasant/palatable taste, *etc.),* the effects of both pH and acid identity on the taste profile of loratadine in liquid formulations were examined. A liquid formulation containing base vehicles and loratadine was prepared (**Table 1A**), and citric acid, fumaric acid, malic acid, phosphoric acid, succinic acid, or tartaric acid solutions were slowly added to the liquid formulations to achieve various pH values. A taste acceptability index (TAI) score was assigned to each of these formulations based on the average score given to each formulation from a panel of tasters **(****FIG. 1****).** Surprisingly, not all acids were found to impact the taste acceptability index of the liquid formulation in the same way at a given pH. For example, at a pH 2.6, little to no bitterness (TAI scores of 7-9) was detected in the liquid formulations containing fumaric acid, malic acid, tartaric acid, or phosphoric acid, but medium to high levels of bitterness (TAI scores of 3-5) were detected at this pH using citric acid or succinic acid. These results suggest that both pH and the identity of the acid used in an API-containing formulation could impact important properties of the formulation (*i.e.,* taste). For example, **FIG. 1** indicates that a formulation containing citric acid and having a pH that ranges from approximately 3.1 to 5 resulted in a high TAI score; that a formulation containing fumaric acid and having a pH that ranges from approximately 2.6 to 5 resulted in a high TAI score; that a formulation containing malic acid and having a pH that ranges from approximately 2 to 5 resulted in a high TAI score; that a formulation containing phosphoric acid and having a pH that ranges from approximately 2 to 5 resulted in a high TAI score; that a formulation containing succinic acid and having a pH that ranges from approximately 3.1 to 5 resulted in a high TAI score; and that a formulation containing tartaric acid and having a pH that ranges from approximately 2.4 to 5 resulted in a high TAI score.

**Table 1A: Liquid formulation containing loratadine**

| **Ingredient:** | **Quantity (% w/w)** |
|---|---|
| Loratadine | 0.217 |
| Propylene glycol | 0.78 |
| Corn syrup 43/43 (80-81% solids) | 41.215 |
| Sucrose | 33.623 |
| Purified water | 24.170 |
| **Total** | 100.004 |

Chewable gel products (as shown in **Table 1B**) were next prepared based upon the above described liquid formulation with the addition of a gel carrier (a mixture containing carrageenan and pectin). The taste of these formulations was assessed, and it was found that the formulations containing either phosphoric acid or citric acid were bitter, while the formulation that did not include an acid has no bitterness. These results are consistent with the data shown in **FIG. 1**. Decreasing the pH of the formulation was found to increase the instability and degradation of the loratadine.

**Table 1B: Chewable gel product formulations containing loratadine**

| | **Formulation-009** | | **Formulation-010** | | **Formulation-011** | |
|---|---|---|---|---|---|---|
| **Ingredient** | **mg/dose** | **% w/w** | **mg/dose** | **% w/w** | **mg/dose** | **% w/w** |
| Loratadine | 20.00 | 0.447 | 20.00 | 0.447 | 20.00 | 0.447 |
| Glycerin | 80.00 | 1.789 | 80.00 | 1.789 | 80.00 | 1.789 |
| Carrageenan-pectin | 77.00 | 1.722 | 77.00 | 1.722 | 77.00 | 1.722 |
| Sucrose | 1704.00 | 38.115 | 1704.00 | 38.115 | 1704.00 | 38.115 |
| Corn Syrup 43/43 (80-81% Solids) | 2018.00 | 45.139 | 2018.00 | 45.139 | 2018.00 | 45.139 |
| FD&C Red No. 40 | 0.65 | 0.015 | 0.65 | 0.015 | 0.65 | 0.015 |
| Mixed Berry Flavor | 11.00 | 0.246 | 11.00 | 0.246 | 11.00 | 0.246 |
| Purified Water | 560.00 | 12.526 | 560.00 | 12.526 | 560.00 | 12.526 |
| Phosphoric Acid 10% Solution | * | | qs.ad. pH 2.98 | | - | - |
| Citric Acid 25% Solution | | | - | - | qs.ad. pH 3.27 | |
| **Total** | 4470.65 | 100.000 | 4470.65 | 100.000 | 4470.65 | 100.000 |

| | | | | | | |
|---|---|---|---|---|---|---|
| *natural pH 4.9 (no acid added) | | | | | | |

The effect of increasing levels of loratadine in liquid formulations was also tested to determine whether higher levels of loratadine led to increased bitterness in the formulations **(Table** 2). However, no difference in taste was observed at any of the concentrations of loratadine that were evaluated. The results indicate that the inherent pH of liquid formulation at four different concentrations of loratadine did not affect bitterness. Thus, the high TAI values in FIG. 1 do not appear to be due to the dilution of the loratadine in the liquid formulation. The results also indicate that lack of bitterness appears to be dependent on maintaining the insolubility of loratadine, independent of concentration. Thus for a particular acid (e.g., citric acid, tartaric acid, etc.), maintaining a pH above a certain level required to solubilize loratadine yielded a product with an acceptable, non-bitter taste. The results also indicate that adjusting pH with different acids yielded different taste profiles. In particular, use of each of the different acids to adjust pH (even if adjusting to the same pH) resulted in a different taste. At a particular pH, a given acid would result in a more bitter product at a same pH than when a different acid was used.

**Table 2: Loratadine concentration effects**

| **Ingredient:** | **Formulation A** | **Formulation B** | **Formulation C** | **Formulation D** |
|---|---|---|---|---|
| Loratadine, % | 0.2 | 0.4 | 0.8 | 1 |
| Corn syrup, % | 38 | 38 | 38 | 38 |
| Sucrose, % | 31 | 31 | 31 | 31 |
| Water, % | 30.8 | 30.6 | 30.2 | 30 |
| **Total, %** | 100 | 100 | 100 | 100 |
| **Total, mg** | 5000 | 2500 | 1250 | 1000 |

The effect of adding various flavorants (mixed berry, mulberry, and menthol) on taste/bitterness was next evaluated **(Table 3).** The addition of the various flavorants did not appear to have any particular effect on the taste/bitterness of the formulation other than the typical masking effect of the flavorants.

**Table 3: Flavorant effects**

| **Ingredient** | **037B** | **037A** | **052** |
|---|---|---|---|
| | **% w/w** | **%w/w** | **% w/w** |
| Loratadine | 0.455 | 0.455 | 0.455 |
| Glycerin | 1.821 | 1.821 | 1.821 |
| Sucrose | 46.000 | 46.000 | 46.000 |
| Corn Syrup 43/43 (80-81% Solids) | 39.000 | 39.000 | 39.000 |
| Purified Water | 12.624 | 12.624 | 12.624 |
| Artificial Berry Flavor | - | - | 0.1-0.4 |
| Artificial Cherry Flavor | 0.1-0.4 | - | - |
| Natural Honey Flavor | - | 0.1-0.6 | - |
| **Total** | 99.900 | 99.900 | 99.900 |

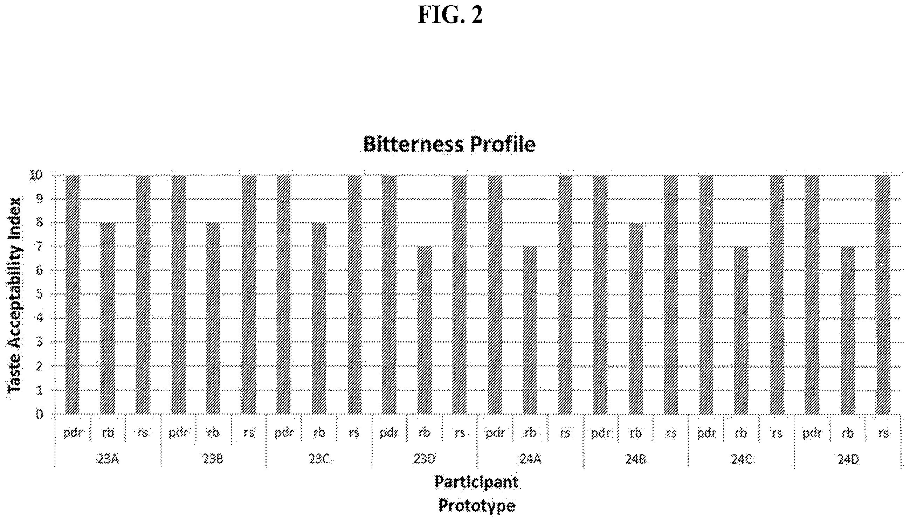

The effect of various sweeteners on taste/bitterness was also evaluated. Formulations were prepared using varying amounts of sweeteners (**Table 4**), and the taste acceptability index for each of the formulations was scored by three tasters: PDR, RB, and RS **(****FIG. 2****).** None of the evaluated sweeteners had any appreciable impact on the bitterness of the loratadine-containing formulations. The results in **Tables 3 and 4** indicate that addition of a flavorant or sweetener does not affect the bitterness of loratadine, and thus reducing the bitterness of loratadine does not appear to depend on the flavorant or sweetener. However, when a flavorant and/or sweetener is combined with maintaining the pH above a certain level required to solubilize loratadine the product yielded an acceptable, non-bitter taste.

**Table 4: Sweetener effects**

| | **Sugar Constant** | | | | **Corn Syrup Constant** | | | |
|---|---|---|---|---|---|---|---|---|
| **Ingredient:** | **23A:** | **23B:** | **23C:** | **23D:** | **24A:** | **24B:** | **24C:** | **24D:** |
| | **% w/w** | **% w/w** | **% w/w** | **% w/w** | **% w/w** | **% w/w** | **% w/w** | **% w/w** |
| Loratadine | 0.46 | 0.46 | 0.46 | 0.46 | 0.46 | 0.46 | 0.46 | 0.46 |
| Glycerin | 1.82 | 1.82 | 1.82 | 1.82 | 1.82 | 1.82 | 1.82 | 1.82 |
| Sucrose | 39.00 | 39.00 | 39.00 | 39.00 | 20.00 | 25.00 | 30.00 | 45.00 |
| Corn syrup 43/43 (80%) | 30.00 | 35.00 | 46.00 | 50.00 | 46.00 | 46.00 | 46.00 | 46.00 |
| Water | 28.47 | 23.47 | 12.47 | 8.47 | 31.47 | 26.47 | 21.47 | 6.47 |
| Mixed berry flavor | 0.25 | 0.25 | 0.25 | 0.25 | 0.25 | 0.25 | 0.25 | 0.25 |
| **Total** | 100 | 100 | 100 | 100 | 100 | 100 | 100 | 100 |

The effect of different gel carriers (gelatin, pectin, and carrageenan), as well as combinations thereof, was then evaluated qualitatively. It was surprisingly found that gel carriers that required a low pH for activation (e.g., gelatin and pectin) appeared to create a chewable gel product that is more bitter in taste than gel carriers that did not require a low pH for activation (e.g., carrageenan), thereby confirming that pH effects required for gelation contributed strongly to the bitter taste profile of the formulations, while no special gel carrier effects contributed to the masking of the bitter taste of loratadine.

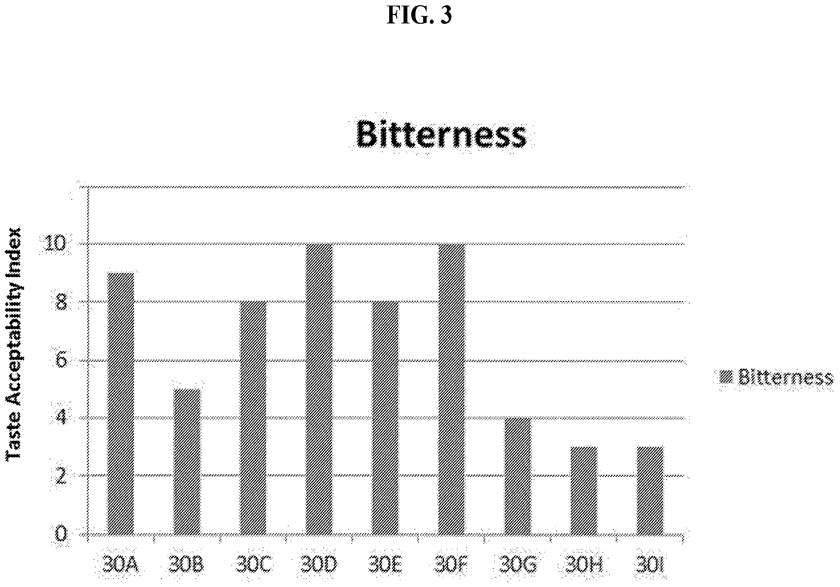

Other active pharmaceutical ingredients (APIs) were tested to assess their taste acceptability index score (**Table 5**). Loratadine, desloratadine, phenylephrine, chlorpheniramine maleate, and dextromethorphan HBr appeared to have acceptable taste acceptability index scores/bitterness profiles **(****FIG. 3****).**

**Table 5: API formulations**

| **Ingredient:** | | **Loratadine** | **Diphenhydramine HCl** | **Desloratadine** | **Phenylephrine HCl** | **Chlorpheniramine maleate** | |
|---|---|---|---|---|---|---|---|
| | | **mg/ unit dose** | | | | | |
| | | 20.0 | 25.0 | 5.0 | 5.0 | 2.0 | |
| | | **Batch number** | | | | | |
| | | **N/A** | **30A** | **30B** | **30C** | **30D** | |
| | | %w/w | | | | | |
| API | | 0.46 | 0.56 | 0.11 | 0.11 | 0.05 | |
| Glycerin | | 1.82 | 1.82 | 1.82 | 1.82 | 1.82 | |
| Base Vehicle: | | | | | | | |
| | Sugar | 39.00 | 39.00 | 39.00 | 39.00 | 39.00 | |
| | Water | 12.40 | 12.40 | | 12.40 | 12.40 | 12.40 |
| | Corn syrup 43/43 (80-81% Solids) | 46.0 | 46.0 | | 46.0 | 46.0 | 46.0 |
| Water | | 0.07 | 0.00 | | 0.42 | 0.42 | 0.48 |
| Artificial Berry Flavor | | 0.25 | 0.25 | | 0.25 | 0.25 | 0.25 |
| **Total** | | **100.00** | **100.03** | | **100.00** | **100.00** | **100.00** |
| | | | | | | | |

| **Ingredient:** | | **Dextromethorphan HBr** | | **Doxylamine succinate** | **Guaifenesin** | **Fexofenadine HCl** | **Docusate sodium** |
|---|---|---|---|---|---|---|---|
| | | **mg/ unit dose** | | | | | |
| | | 10.00 | | 6.25 | 200.00 | 60.00 | 50.00 |
| | | **Batch number** | | | | | |
| | | **30E** | | **30F** | **30G** | **30H** | **30I** |
| | | % w/w | | | | | |
| API | | 0.22 | | 0.14 | 4.30 | 1.33 | 1.11 |
| Glvcerin | | 1.82 | | 1.82 | 1.82 | 1.82 | 1.82 |
| Base Vehicle: | | | | | | | |
| | Sugar | 39.00 | | 39.00 | 39.00 | 39.00 | 39.00 |
| | Water | 12.40 | | 12.40 | 12.40 | 12.40 | 12.40 |
| | Corn syrup 43/43 (80-81% Solids) | 46.0 | | 46.0 | 46.0 | 46.0 | 46.0 |
| Water | | 0.31 | | 0.39 | 0.00 | 0.00 | 0.0 |
| Artificial Berry Flavor | | 0.25 | | 0.25 | 0.25 | 0.25 | 0.25 |
| **Total** | | **100.00** | | **100.00** | **103.77** | **100.80** | **100.58** |

Finally, a sugar-free carrageenan-pectin-based formulation **(Table 6)** and a sugar-free carrageenan-based formulation **(Table 7)** were developed. The two sugar-free formulations were tested for their ability to maintain stability of loratadine. Initially stability was tested using the accelerated stability assessment program (ASAP) for both formulations. Subsequently the stability of the sugar-free carrageenan-based formulation was tested using International Conference on Harmonisation (ICH) conditions for up to six months. Loratadine was found to be stable in both formulations at the conditions tested (data not shown).

**Table 6: Sugar-free carrageenan-pectin-based formulation**

| **Ingredient:** | **% w/w** |
|---|---|
| Loratadine | 0.500 |
| Propylene glycol | 1.500 |
| Carrageenan-pectin | 2.125 |
| Maltitol solids | 77.460 |
| Colorant (FD&C Red 40) | 0.015 |
| Artificial Berry Flavor | 0.800 |
| Purified water | 17.600 |
| **Total** | **100.00** |

**Table 7: Sugar-free carrageenan-based formulation**

| **Ingredient:** | **%w/w** |
|---|---|
| Loratadine | 0.500 |
| Propylene glycol | 1.500 |
| Carrageenan | 2.125 |
| Maltitol solids | 76.760 |
| Colorant (FD&C Red 40) | 0.015 |
| Artificial Berry Flavor | 0.400 |
| Aspartame | 0.200 |
| Purified water | 18.300 |
| Sucralose | 0.200 |
| **Total** | **100.00** |

Taken together, this data suggests that there appears to be a surprising effect of both pH and the acid type on the bitterness of chewable gel products. Additionally, there appears to be an improvement in stability of the API at higher pH. API concentration, flavorants, sweeteners, and gel carriers were not observed to have an effect on the bitterness of the API. Without wishing to be bound by theory, it is believed that regardless of what gel carrier is used, they should fall within a particular range to provide a chewable gel product with good taste (*e.g*., no bitterness or unpalatable taste profile) and acceptable stability of the active pharmaceutical ingredient(s).

### Example 2: Pectin-based loratadine chewable gel product

The following example describes formulations of pectin-based chewable gel products containing loratadine.

**Table 8A: Pectin-based chewable gel formulation**

| **Ingredient:** | **% w/w** |
|---|---|
| Loratadine | 0.500 |
| Propylene glycol | 1.500 |
| Pectin | 1.2-2.0 |
| Maltitol solids | 77.460 |
| Colorant FD&C Red 40 | q.s. |
| Artificial Berry Flavor | q.s. |
| Sucralose | 0.300 |
| Fumaric acid | q.s. pH 3.5 |
| Purified water | q.s. 100 |
| **Total** | **100.00** |

**Table 8B: Pectin-based chewable gel formulation**

| **Ingredient:** | **% w/w** |
|---|---|
| Loratadine | 0.500 |
| Propylene glycol | 3.000 |
| Pectin | 1.800 |
| Maltitol | 75.500 |
| Colorant FD&C Red 40 | 0.015 |
| Mixed berry flavor | 0.800 |
| Citric acid* | 0.800 |
| Purified water | 17.585 |
| **Total** | **100.00** |

| | |
|---|---|
| *The citric acid is added to adjust pH to 3.2-3.6 to yield a suitable firmness | |

### Example 3: Gelatin-based loratadine chewable gel product

The following example describes formulations of gelatin-based chewable gel products containing loratadine.

**Table 9A: Gelatin-based chewable gel formulation**

| **Ingredient:** | **% w/w** |
|---|---|
| Loratadine | 0.500 |
| Propylene glycol | 1.500 |
| Gelatin | 5-7 |
| Maltitol solids | 76.760 |
| Colorant FD&C Red 40 | q.s. |
| Artificial Berry Flavor | q.s. |
| Aspartame | 0.200 |
| Sucralose | 0.200 |
| Tartaric acid | q.s. pH 3.2-3.8 |
| Purified water | q.s. 100 |
| **Total** | **100.00** |

**Table 9A: Gelatin-based chewable gel formulation**

| **Ingredient:** | **% w/w** |
|---|---|
| Loratadine | 0.500 |
| Glycerin | 3.000 |
| Gelatin | 5.500 |
| Sucrose | 38.000 |
| Corn symp | 45.000 |
| Colorant FD&C Red 40 | 0.015 |
| Mixed berry flavor | 0.800 |
| Citric acid* | 0.800 |
| Purified water | 6.385 |
| **Total** | **100.00** |

| | |
|---|---|
| *The citric acid is added to adjust pH to 3.2-4.5 to yield a suitable firmness | |

### Example 4: Carrageenan-based loratadine chewable gel product

The following example describes formulations of carrageenan-based chewable gel products containing loratadine.

**Table 10: Carrageenan-based chewable gel formulation**

| | **Formulation 10A** | | **Formulation 10B** | |
|---|---|---|---|---|
| **Ingredient:** | **mg/dose** | **% w/w** | **mg/dose** | **% w/w** |
| Loratadine | 10.00 | 0.500 | 10.00 | 0.500 |
| Sorbitol | 1162.96 | 58.148 | 1017.59 | 50.880 |
| Maltitol | 290.74 | 14.537 | 436.11 | 21.806 |
| Purified water | 420.00 | 21.000 | 420.00 | 21.000 |
| Propylene glycol | 60.00 | 3.000 | 60.00 | 3.000 |
| Carrageenan | 36.00 | 1.800 | 36.00 | 1.800 |
| Mulberry flavor | 8.00 | 0.400 | 8.00 | 0.400 |
| Aspartame | 4.00 | 0.200 | 4.00 | 0.200 |
| Potassium citrate | 4.00 | 0.200 | 4.00 | 0.200 |
| Sucralose | 4.00 | 0.200 | 4.00 | 0.200 |
| FD&C Red 40 | 0.30 | 0.015 | 0.30 | 0.015 |
| MCT coconut oil with carnauba wax | Trace | Trace | Trace | Trace |
| **Total** | **2000.00** | **100.00** | **2000.00** | **100.00** |

In **Table 10,** the MCT coconut oil with carnauba wax is used as an anti-sticking agent that also provides a glossy coating to the product. The MCT coconut oil with carnauba wax is a blend of coconut oil and carnauba wax.

## Claims

1. A method for producing a chewable gel product, comprising:
a. formulating a liquid composition comprising a gel carrier and an active pharmaceutical ingredient;
b. adjusting the pH of the liquid composition to a pH that ranges from about 2.5 to about 8; and
c. gelating the liquid composition at a temperature sufficient to yield a chewable gel product.

2. The method of claim 1, wherein the gel carrier is a non-acid-activated gel carrier.

3. The method of claim 2, wherein the non-acid-activated gel carrier comprises carrageenan, a combination of carrageenan and pectin, or a combination of carrageenan and gelatin.

4. The method of claim 1, wherein the gel carrier is an acid-activated gel carrier.

5. The method of claim 4, wherein the acid-activated gel carrier comprises pectin or gelatin.

6. The method of claim 4 or claim 5, further comprising adding a taste-compatible acid to the liquid composition.

7. The method of claim 6, wherein the taste-compatible acid is one or more acids selected from the group consisting of citric acid, fumaric acid, malic acid, phosphoric acid, succinic acid, tartaric acid, maleic acid, acetic acid, hydrochloric acid, lactic acid, propionic acid, salts thereof, and derivatives thereof.

8. The method of claim 6 or claim 7, wherein the taste-compatible acid is added in an amount that yields a pH value that ranges from 2.5 to 4.0.

9. The method of any one of claims 1 to 8, wherein the active pharmaceutical ingredient is selected from the group consisting of loratadine, diphenhydramine, desloratadine, phenylephrine, chlorpheniramine, dextromethorphan, doxylamine, guaifenesin, fexofenadine, docusate, pseudoephedrine, cetirizine, triprolidine, brompheniramine, ephedrine, ibuprofen, acetaminophen (paracetamol), ketoprofen, naproxen, piroxicam, meloxicam, leflunomide, ondansetron, granisetron, carbamazepine, lamotrigine, clozapine, olanzapine, risperidone, citalopram, paroxetine, sertraline, fluoxetine, fluvoxamine, zopiclon, zolpidem, cimetidine, ranitidine, omeprazole, metoclopramide, cisapride, domperidon, zafirlukast, montelukast, pramipexol, selegiline, doxazosin, terazosin, atenolol, bisoprolol, amlodipine, nifedipine, diltiazem, enalapril, captopril, ramipril, losartan, glyceroltrinitrate, alfuzosin, finasteride, pravastatin, atorvastatin, simvastatin, gemfibrozil, metformin, terfenadine, celecoxib, rifecoxib, rivastignine, astemizole, hydroxyzine, clemastine, local anesthestics, antiseptics, opioids, opioid derivatives, sildenafil, tadalafil, vardenafil, and a pharmaceutically acceptable salt, ester, hydrate or solvate thereof.
